# EUROPEAN PATENT APPLICATION

(11) **EP 1 571 210 A1**
(43) Date of publication of application: **07.09.2005**
(21) Application number: 03778789.2
(22) Date of filing: 10.12.2003
(51) Int. Cl.: C12N 15/09, C12Q 1/68, C12M 1/00, G01N 33/53, G01N 33/50

(54) **METHOD FOR ANALYZING VARIATION OF NUCLEIC ACID MUTATION AND METHOD FOR ANALYZING GENE EXPRESSION**

(30) Priority: 10.12.2002 JP 2002358504; 10.12.2002 JP 2002358505; 08.12.2003 JP 2003408945
(71) Applicant: Olympus Corporation, Tokyo 151-0072 (JP)
(72) Inventor: TAKAHASHI, Takeo, Ota-ku, Tokyo 143-0014 (JP)
(74) Representative: von Hellfeld, Axel
(86) International application number: PCT/JP2003/015819
(87) International publication number: WO 2004/061100

(57) **Abstract**

A method for determining the type of a base at a target site in a target nucleic acid, comprising a step of reacting a target nucleic acid sample with a probe nucleic acid which contains a selection sequence complementary to the nucleotide sequence located on the 3' side adjacent to the target site of the target nucleic acid and which is immobilized on a substrate via its 5' end, a step of elongating a reaction product obtained in the above step in the presence of both a polymerase and a labeled deoxyribonucleoside triphosphate containing a specific one type of base labeled with a marker substance generating a detectable signal, a step of removing the labeled deoxyribonucleoside triphosphate which has not been used in the elongation step from the reaction system, a step of detecting a signal derived from the marker substance present in the reaction system, and a step of determining the type of a base at the target site based on the presence or absence of the detection of the signal and on the type of the base contained in the labeled deoxyribonucleoside triphosphate.

## Description

### Technical Field

The present invention relates to a method for analyzing a variation of a nucleic acid using an immobilized probe nucleic acid. The invention also relates to a method for analyzing a gene expression using an immobilized labeled probe nucleic acid. The invention further relates to a method for preparing a labeled probe nucleic acid useful in various analyses using nucleic acids, for example a gene expression analysis.

### Background Art

A so-called DNA microarray is a device having a number of different probe DNAs immobilized at a high density on a solid phase substrate such as a glass. Such a device having a probe nucleic acid on a substrate is classified broadly into two types based on the production method. One is a device involving a synthesis of a DNA on a glass surface by a photolithographic method, i.e., a device generally referred to as a DNA chip (Proc. Natl. Acad. Sci. USA (1994) 91: 5022-5026). The other is a device involving a mechanical alignment of a previously prepared DNA on a glass slide whereby attaching the DNA on it, i.e. a device generally referred to as a DNA microarray (Science (1994) 270: 467-470).

The fundamental measurement principle of the two types of the DNA microarrays described above is that a labeled nucleic acid sample is hybridized with an immobilized probe nucleic acid and then whether a nucleic acid hybridizing with the probe nucleic acid is present or not in the nucleic acid sample is detected based on the label of the nucleic acid sample. By means of such a principle, a massive analysis of genes becomes possible. In addition, an improved detection sensitivity, a saving of sample due to a reduction of the device size, an automated data acquisition and a simplified data processing are also expected to be achieved.

A DNA microarray of the type involving a synthesis of a DNA on a glass surface is made by combining a photolithographic technology employed for a semiconductor with a solid phase DNA synthesis technology. First, a synthetic linker having a protective group cleavable by a photoreaction is bound on a glass substrate, which is irradiated with a light via a shield material referred to as a mask whereby cleaving the protective group in a certain region. Then, the glass substrate is reacted with a nucleotide whose hydroxyl group is protected. As a result, a polymerization reaction occurs only in a part where the protective group has been cleaved off. Then the light is irradiated onto a different region on the substrate using another mask to repeat the nucleotide polymerization whereby making an array.

A DNA microarray of the type involving an attachment of a DNA onto a glass slide does not need a large-scale semiconductor producing machine. Only with a DNA array machine and a detector, an intended DNA microarray can be produced. While this method is advantageous since a DNA to be attached can arbitrarily be selected, it requires a preparation of a DNA collection which requires a complicated work. Also this method involves a physical spotting by a pin tip. Accordingly, the DNA density is not so high as one achieved by a photolithographic method. However, it is possible theoretically to obtain 2500 spots per 1 cm² if a spot whose diameter is 100 µm is spotted at a 100 µm interval. Per ordinary glass slide (about 4 cm² as a valid surface area), about 10,000 DNAs can be mounted.

A DNA array machine employs a combination of high performance servomotors to transport a DNA sample onto the surface of a glass slide from a microtitre plate by operating a pin tip or a slide holder in an XYZ direction under control by a computer. The pin tip shape is very sophisticated and serves as a pivot of this technology. The most ordinary pin tip shape is a divided pen tip shape like a drafting pen. There a DNA probe solution is retained and spotted on a plurality of slides. A step of mounting a subsequent DNA sample is repeated via a washing and drying cycle. A function required for an ideal array machine is a uniform spot size or shape together with a high speed processing and a high reproducibility. Since the uniformity and the speed are limited for example by difference in the pen tip lot, a novel technology such as an ink jet method or capillary method is subjected to an ongoing development for the purpose of obtaining a further higher performance.

With regard to a DNA immobilization method, various coatings are attempted to be used since a glass has a smaller valid immobilization area and a lower electric charge when compared with a membrane. Practically, a poly-L lysine or silanization are employed. In a method, a DNA terminal is aminated and then crosslinked with a silanized glass.

The detection of a fluorescent signal of a fluorescent labeled DNA hybridized on a DNA microarray is conducted by a fluorescence detector. Usually, a scanner is a combination of a fluorescence microscope and a movable stage. Even with a conventional fluorescent image analyzer for a gel, a DNA microarray whose density is not so high can be read. Recently, a high resolution scanner exclusive for a DNA microarray was marketed. It is classified into a confocal type or an incident-light type on the basis of the readout mode.

Since the DNA microarray output data is of a great size, a data analysis software is regarded as important. A software used easily by an operator in terms of the data display mode, data post-processing and link to other database is desired to be developed. An attempt to construct a database from a gene expression monitoring by a microarray or an internet publication of an array database are also started.

By using a DNA microarray of the technology described above, detection of a mutation has been attempted. First, four types of oligonucleotide probes, which are different only in the single base corresponding to a single base (mutation) in a certain region of a sample, are designed and mounted on a DNA probe chip. On the other hand, the sample whose mutation is to be detected is amplified by a PCR and the terminal is labeled using a fluorescent dye. When this sample is hybridized with a DNA chip described above, a probe having a nucleotide sequence complementary to the sample sequence exhibits a most intense hybridization signal. Similarly, in the case of a sample having a mutation of a single base substitution, a probe having a base complementary to the mutated base exhibits a most intense hybridization signal. While this method can not determine an unknown nucleotide sequence unlike a DNA sequencing based on an electrophoresis, the presence of a mutation in a predetermined region of a certain gene can be detected at a high density.

In preparing a sample, for example, for a Hu SNP Mapping Assay (Affymetrix), a multiple PCR is employed for the amplification of an SNP marker, which amplifies about 100 types of the PCR products in a single reaction tube. On the second stage, a labeling PCR is conducted, thereby effecting a biotin labeling on a PCR product containing each SNP marker while further amplification, wherein a primer pair which is common to all PCR products can be used. By means of such an efficient sample preparation, it became possible to determine an SNP gene type within one and a half day starting from about 120 ng of a genome DNA. The experimental procedure is automated ultimately by a GeneChip system, which enables an analysis of 180 samples within 24 hours when employing one scanner.

In such a DNA variation analysis, 4 types of oligonucleotide probes are immobilized on a solid phase substrate such as glass, on which a PCR-amplified fluorescent labeled DNA is hybridized and a signal from each probe is detected by an automatic detector, whose data are analyzed by a computer. Such an analysis involves the following problems. Specifically, 1) a sample to be determined should be labeled on each sample basis using a fluorescent labeled nucleotide or a fluorescent labeled primer, thus posing a poor labeling efficiency and a high production cost, 2) a pretreatment before the hybridization is complicated and difficult to be automated, 3) a determination of a single sample requires 4 types of probes which requires a substantial expense, and 4) the efficiency of fluorescent signal detection is low since the determination is conducted in a dry condition.

On the other hand, a gene expression analysis using a DNA microarray described above is also conducted currently. It mainly employs a system observing a differential gene expression using a double fluorescent labeling method. In principle, it detects a difference in a gene expression between two different mRNA samples. For this purpose, upon preparation of each labeled cDNA from each mRNA sample, each cDNA is labeled with a different fluorescent substance, and each labeled cDNA is hybridized competitively with a probe on the array, and the both fluorescents are measured and compared.

Such a conventional method involves the following problems. Specifically, 1) a conventional method involves a labeling of a sample to be determined which may cause a degradation of the sample such as an RNA. 2) A conventional method is difficult to be automated since a pretreatment before the hybridization is complicated. 3) A conventional method detects a differential gene expression, which allows the expression level to be observed only as a relative change. 4) A conventional method poses a difficulty in knowing the immobilized probe amount or the deposited spot condition before a reaction, which makes it difficult to control the accuracy of probe immobilization. 5) A conventional method suffers from a low efficiency of the fluorescent signal detection because of the measurement in a dry condition.

### Disclosure of Invention

Under the circumstance described above, an object of the invention is to provide a method for analyzing a gene variation which is highly efficient and economically advantageous.

As a result of substantial studies, the inventor found a means for solving the problems described above. Specifically, the means is as follows:
A method for determining the type of a base at a target site in a target nucleic acid, comprising:
   (1) a step of reacting a nucleic acid sample with a probe nucleic acid, which contains a selection sequence complementary to the nucleotide sequence located on the 3' side adjacent to the target site of the target nucleic acid and which is immobilized on a substrate via its 5' end, in a reaction system under a condition for achieving an appropriate hybridization;
   (2) a step of elongating a reaction product obtained in the step (1), in the presence of both a polymerase and a labeled deoxyribonucleoside triphosphate containing a specific one type of base labeled with a marker substance generating a detectable signal, under a condition for obtaining an appropriate elongation reaction;
   (3) a step of removing the labeled deoxyribonucleoside triphosphate which has not been used for the elongation reaction in the elongation step (2) from the reaction system;
   (4) a step of detecting a signal derived from the marker substance present in the reaction system after the step (3); and
   (5) a step of determining the type of a base at the target site based on the presence or absence of the detection of the signal in the step (4) and on the type of the base contained in the labeled deoxyribonucleoside triphosphate.

Also under the circumstance described in the section of the background art, an object of the invention is to provide an efficient method for analyzing a gene expression. A further object of the invention is to provide a method for analyzing a gene expression which is capable of comparing the gene expression level between a plurality of plates. A still further object of the invention is to provide a method for analyzing a gene expression having a high detection accuracy.

As a result of substantial studies, the inventor found a means for solving the problems described above. Specifically, the means is as follows:
A method for detecting the presence of a target sequence in a test nucleic acid, comprising:
   (1) a step of reacting the test nucleic acid with a labeled probe nucleic acid, which is immobilized onto a channel allowing the reaction to be conducted there and which has a marker substance attached thereto, under a condition for enabling an appropriate hybridization reaction;
   (2) a step of adding, after the step (1), a single-stranded specific nuclease to the channel to conduct a reaction under a condition for allowing an appropriate enzyme reaction to be achieved;
   (3) a step of removing, after the step (2), a degradation product of the enzyme reaction from the channel;
   (4) a step of detecting a signal from the marker substance contained in the double-stranded nucleic acid in the channel; and
   (5) a step of detecting the presence of the target sequence in the test nucleic acid based on a signal detected in the step (4).

Also provided is a method for determining the expression frequency of a target sequence in a subject, comprising:
(1) a step of reacting a sample containing a test nucleic acid derived from a subject with a labeled probe nucleic acid, which is immobilized onto a channel allowing the reaction to be conducted there and which has a marker substance attached thereto, under a condition for enabling an appropriate hybridization reaction;
(2) a step of adding, after the step (1), a single-stranded specific nuclease to the channel to conduct a reaction under a condition for allowing an appropriate enzyme reaction to be achieved;
(3) a step of removing, after the step (2), a degradation product of the enzyme reaction from the channel;
(4) a step of detecting, after the step (3), a signal derived from the marker substance contained in the double-stranded nucleic acid in the channel; and
(5) a step of determining the expression frequency of the target sequence in the subject based on a signal detected in the step (4).

Also upon conducting such a gene expression analysis method, the inventor found that this method requires a substantial expense, time and effort since it requires labeling all probes on a support such as a channel.

Accordingly, an object of the invention is to provide a convenient method for labeling a probe which is capable of labeling all probes on an array at once. Specifically, the object of the invention is to provide a method for easily making a support having a labeled probe nucleic acid immobilized thereon.

In order to solve the problems described above, the invention provides the following means:
A method for preparing a support having a labeled probe nucleic acid immobilized thereon, comprising:
   (1) a step of reacting between a template nucleic acid complementary to the nucleotide sequence of a labeled probe nucleic acid to be prepared and an unlabeled probe nucleic acid precursor which is immobilized on a substrate via its 5' end, under a condition for allowing them to be hybridized, wherein the unlabeled probe nucleic acid precursor has a fewer total number of the bases than the template nucleic acid and has a deletion of a part of the nucleotide sequence of the labeled probe nucleic acid to be prepared;
   (2) a step of elongating the deficient part of the unlabeled probe nucleic acid precursor in the direction from 5' to 3', using both a labeled nucleotide of a specific base to which a marker substance generating a detectable signal is attached and non-labeled nucleotides of bases other than the specific base as substrates and using the template nucleic acid as a template, whereby synthesizing a labeled probe nucleic acid;
   (3) a step of dissociating all complementary bonds between the labeled probe nucleic acid obtained in the above step and the template nucleic acid; and
   (4) a step of removing the dissociated template nucleic acid from the support.

### Brief Description of Drawings

FIG. 1 shows an example of a reaction vessel used according to one embodiment of the invention; FIG. 1(a) is a plan view of an example of a reaction vessel used according to one embodiment of the invention; FIGS. 1(b) and 1(c) are sectional views at a line 1B-1B in FIG. 1(a);
FIG. 2 is a schematic view of an example of a variation analysis method according to one embodiment of the invention;
FIG. 3 is a schematic view of an example of a variation analysis method according to one embodiment of the invention;
FIG. 4 is a schematic view of an example of a nucleic acid degradation by an S1 nuclease;
FIG. 5 is a schematic view of the principle of a nuclease protection assay;
FIG. 6 is a schematic view of an example of a variation analysis method according to one embodiment of the invention;
FIG. 7 is a schematic view of an example of a variation analysis method according to one embodiment of the invention;
FIG. 8 is a schematic view of an example of a gene expression analysis method according to one embodiment of the invention;
FIG. 9 is a schematic view of an example of a method for labeling a probe nucleic acid according to one embodiment of the invention;
FIG. 10 is a schematic view of an example of a method for detecting a target sequence according to one embodiment of the invention;
FIG. 11 is a schematic view of an example of a method for labeling a probe nucleic acid according to one embodiment of the invention;
FIG. 12 is a schematic view of an example of a method for labeling a probe nucleic acid according to one embodiment of the invention;
FIG. 13 is a schematic view of an example of a method for labeling a probe nucleic acid according to one embodiment of the invention;
FIG. 14 is a schematic view illustrating a first embodiment of a method for producing a support having a labeled probe nucleic acid immobilized thereon according to the invention;
FIG. 15 is a schematic view illustrating a second embodiment of a method for producing a support having a labeled probe nucleic acid immobilized thereon according to the invention;
FIG. 16 is a schematic view illustrating a third embodiment of a method for producing a support having a labeled probe nucleic acid immobilized thereon according to the invention; and
FIG. 17 is a photomicrograph showing the results of the 16th example.

The reference numerals employed in the figures are as follows:
1: reaction vessel, 2: reaction zone, 3: probe nucleic acid, 4a, 4b: opening, 5: polyimide thin film, 6: heater, 7: temperature sensor;
21a, 21b: target nucleic acid, 22a, 22b: probe nucleic acid, 23a, 23b: labeled substrate nucleic acid;
31a, 31b, 31c: target nucleic acid, 32a, 32b, 32c: probe nucleic acid, 33: labeled substrate nucleic acid, 34: reaction zone bottom, 35: reaction zone, 36a, 36b, 36c: elongated labeled probe nucleic acid;
81a, 81b, 81c: target nucleic acid, 82a, 82b, 83c: probe nucleic acid, 83: labeled substrate nucleic acid, 84: reaction zone bottom, 85: reaction zone, 86a, 86b, 86c: elongated labeled probe nucleic acid, 87a, 87b: target nucleic acid;
91a: target nucleic acid, 92: probe nucleic acid, 93a: labeled substrate nucleic acid, 94: reaction zone bottom, 95: reaction zone, 96a: elongated labeled probe nucleic acid, 91b: target nucleic acid, 93b: labeled substrate nucleic acid, 96b: elongated labeled probe nucleic acid;
101: probe nucleic acid, 102: target nucleic acid, 104: reaction zone bottom, 105: reaction zone;
121: template nucleic acid, 122: seed probe nucleic acid, 123: labeled substrate nucleic acid, 124: reaction zone bottom, 125: reaction zone, 126: elongated labeled probe nucleic acid, 127, 128: non-labeled substrate nucleic acid;
131a, 131b, 131c: template nucleic acid, 132a, 132b, 132c: seed probe nucleic acid, 133: labeled substrate nucleic acid, 134: reaction zone bottom, 135: reaction zone, 136a, 136b, 136c: elongated labeled probe nucleic acid;
141a: template nucleic acid, 142: seed probe nucleic acid, 143a, 143b: labeled substrate nucleic acid, 144: reaction zone bottom, 145: reaction zone, 146a, 146b: elongated labeled probe nucleic acid;
151: seed probe nucleic acid, 152: reaction zone bottom, 153: template nucleic acid, 154: first labeled substrate nucleic acid, 155: second labeled substrate nucleic acid;
161a, 161b, 161c: template nucleic acid, 162a, 162b, 162c: seed probe nucleic acid, 163: labeled substrate nucleic acid, 164: reaction zone bottom, 165: reaction zone, 166a, 166b, 166c: probe nucleic acid, 167a, 167b: target nucleic acid;
201: support, 202: template nucleic acid, 203: unlabeled probe nucleic acid precursor, 204: labeled nucleotide, 205: non-labeled nucleotide, 206: labeled probe nucleic acid;
207: dideoxynucleotide;
203a: unlabeled probe nucleic acid precursor A, 203b: unlabeled probe nucleic acid precursor B.

### Best Mode for Carrying Out the Invention

### 1. Nucleic acid variation analysis method

### 1.1 Outlines of nucleic acid variation analysis method

According to an embodiment of the invention, a method for determining the type of a base at a specific site in a target nucleic acid is provided. In this method, a probe nucleic acid which contains a sequence for capturing a target nucleic acid selectively and which is immobilized at its 5' end onto a substrate is employed.

As used herein, the term "nucleic acid" means any of various naturally occurring DNAs and RNAs, as well as artificially synthesized nucleic acid analogues such as peptide nucleic acids, morpholino nucleic acids, methylsulfonate nucleic acids and S-oligo nucleic acids and the like.

As used herein, a "target nucleic acid" means any of the nucleic acids described above which is a nucleic acid having a base length appropriate for hybridization. A base length appropriate for hybridization may be 10-base length to 1000-base length, preferably 15-base length to 200-base length, more preferably 15-base length to 50-base length.

For example, a preferable target nucleic acid employed as a subject according to the invention may be any of nucleic acid fragments such as a DNA fragment, RNA fragment, polynucleotide, oligonucleotide and the like. Such a target nucleic acid may also be an artificially synthesized nucleic acid fragment, any nucleic acid fragment extracted from a subject, a cDNA or RNA fragment synthesized therefrom, as well as a nucleic acid fragment obtained for example by a PCR amplification. It may also be a nucleic acid fragment obtained by an operation using a certain cell engineering technology known per se.

As used herein, the term "subject" means any animal such as mammals including human, monkey, cattle, pig, sheep, goat, dog, cat, rabbit, rat and mouse, and reptiles, fishes and insects, as well as other living individuals capable of expressing genes, as well as a cell and a tissue obtained from any individuals.

A step of obtaining a nucleic acid sample containing a target nucleic acid from a subject described above can be conducted by a means known per se. For example, when a nucleic acid is a genome DNA, then a commercially available kit may be used or other known method may be used. Also for example, when a nucleic acid sample is an RNA, a commercially available kit may be used or an oligo dT column may be used.

A "probe nucleic acid" used in a method of the present invention is a nucleic acid for selectively capturing a "target nucleic acid" having a specific sequence by utilizing hybridization. Accordingly, the probe nucleic acid contains, as a selection sequence, a sequence complementary to a part which is consecutive in an already known sequence of a target nucleic acid to be analyzed. The length of the probe nucleic acid may be of 10 bases to 100 bases, preferably 15 bases to 25 bases. The length of the selection sequence may be of 10 bases to 50 bases, preferably 15 bases to 30 bases. The probe nucleic acid is immobilized on a substrate via the 5' end.

The "target nucleic acid" used in a method of the present invention is a sequence to be analyzed in the method, and it is preferable that the sequence of the target nucleic acid is already known except for the target site at which a base type is to be determined. On the 3' side of the target site contained in a target nucleic acid, a sequence complementary to the "selection sequence" mentioned above is present. While the length of a target site may be of 1 base or 2 bases or more, it is preferably of one base. When a probe nucleic acid is immobilized on a substrate via its 5' end and has a selection sequence, a complementary sequence to the selection sequence is located at the 3' side of a target nucleic acid. In this case, the position of a target site in the target nucleic acid may be located adjacent to the complementary sequence to the selection sequence on the 5' side of this complementary sequence. The target site may be on the 5' end of the target nucleic acid or may allow several bases to exist before the end.

A method according to an embodiment of the invention can be conducted for example by a procedure described below. First, a probe nucleic acid having a selection sequence is immobilized on a reaction vessel. Then, the nucleic acid sample is added to the reaction vessel and reacted under a condition for enabling an appropriate hybridization, for example a stringent condition. If a target nucleic acid having a sequence complementary to the selection sequence exists in the nucleic acid sample described above, then the target nucleic acid hybridizes with the probe nucleic acid. As a result, the target nucleic acid having a specific sequence can be captured from the nucleic acid sample. Subsequently, both a polymerase and a labeled deoxyribonucleotide triphosphate having a specific single type base which is labeled with a marker substance generating a detectable signal are added to the reaction vessel, and subjected to an elongation reaction under a condition capable of achieving an appropriate elongation reaction. Thereafter, the labeled substrate which was not used in the elongation reaction is removed, and the detection of the signal derived from the marker substance is carried out. As a result, if such a signal is detected, the elongation is proven to occur. Accordingly, the base on the target site is proven to be a base complementary to the base contained in the labeled deoxyribonucleotide triphosphate employed in the elongation. A single nucleic acid sample may be subjected to such a procedure with regard to all of the 4 types of the base. Also when the target site is a site where a gene polymorphism is present, the procedure mentioned above may be conducted only with regard to the base type possibly constituting the polymorphic gene type.

In an embodiment of the invention, a probe nucleic acid is immobilized on a substrate via its 5' end. The immobilization of the probe nucleic acid can be conducted by immobilizing a desired probe nucleic acid on any of known substrates.

The "substrate" that can be used according to an embodiment of the invention may be in any form allowing a hybridization reaction to be conducted there. For example, it may be a generally employed reaction vessel, including a reaction vessel having a reaction zone for conducting a reaction therein such as a capillary or well, or may be a plate or spherical substrate for conducting the reaction on its surface. Alternatively, the "substrate" may be a needle, strand, fiber, disc or porous filter. Because of an easy handling, a reaction vessel having a reaction zone in the form of a capillary is preferred.

In the case of a spherical substrate, such a spherical substrate can be used to conduct the present invention as described below. Specifically, a reaction of the invention can be conducted by preparing a microparticle on which a plurality of probes corresponding to a target to be detected are immobilized, and then supplying this microparticle into a reaction vessel such as a capillary or a well. Such a microparticle may be recovered from the reaction vessel after the reaction and applied to a known measurement using a flow cytometer (FCM) or laser scanning meter (LSM). When using a microparticle as a substrate, a measurement can be conducted, preferably in another place of a channel connected to a capillary channel of a reaction zone, in a flowing state or a static state. It is also possible to transfer the microparticle sequentially to a different place in a single channel to conduct, at each position, a series of an immobilization step, reaction step and measurement step all at once or consecutively. Since the microparticle is transferred or stopped independently of the liquid in the capillary, each microparticle preferably contains a magnetic material which allows a known magnetic separation technology to be utilized. As mentioned above, the microparticle is transferred in the capillary, and thereby different types of microparticles can be transferred or stopped in good order on the target type basis.
Thus, a microparticle having a certain type of a probe immobilized thereon is allowed to pass through a capillary and then a microparticle having another type of a probe immobilized thereon is allowed to pass through the capillary, whereby different reactions can be carried out in the upstream and the downstream of the capillary channel, respectively. Alternatively, each of the samples containing respective targets is allowed to pass through the capillary channel sequentially, whereby different reactions can be carried out in the upstream and the downstream of the capillary channel, respectively.

As used herein, the term "marker substance" for labeling a nucleotide that is used during an elongation reaction means a substance capable of generating a detectable signal, for example, fluorescent substance, radioactive substance and chemiluminescent substance. A substance developing a color upon an enzyme reaction may also be employed. As in the case where more than one type of probe nucleic acids are employed on a single substrate, more than one type of distinguishable marker substances may simultaneously be employed if desired.

### 1.2 Examples

The invention is further described referring to Examples of the invention.

### Example 1: Reaction vessel

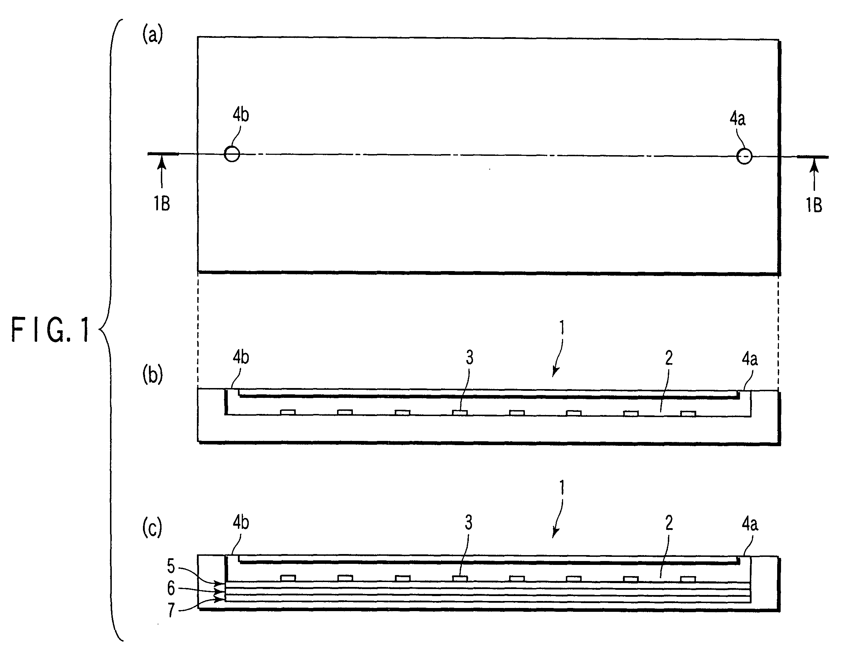

An example of a reaction vessel which can be employed in an embodiment of the invention is shown in FIG. 1. FIG. 1(a) is a plan view of such a reaction vessel, and FIG. 1(b) is a sectional view at a line 1B-1B in FIG. 1(a). A reaction vessel 1 in this Example has a reaction zone 2 to conduct a reaction therein (FIG. 1(b)). The reaction zone 2 has a capillary-shape in its internal structure as shown in FIG. 1(b).

Also as shown in FIGS. 1(a) and 1(b), the reaction vessel 1 has openings 4a and 4b for allowing a reagent and the like to be charged into the reaction zone 2 and/or discharged from the reaction zone 2. On the bottom of the reaction zone 2, a probe nucleic acid 3 containing a selection sequence for capturing a target sequence containing a specific sequence is immobilized on a desired region (FIG. 1(b)). Such a reaction vessel 1 was produced as described below.

A first substrate (capillary cover) having a groove capable of forming a capillary and holes on the both sides of this groove and a second substrate having a probe nucleic acid immobilized thereon are bound to each other, whereby producing a reaction vessel. In this production, the probe nucleic acid is contained in a space formed by the groove of the first substrate and the surface of the second substrate. A more typical description is as follows. While FIG. 1 shows a reaction vessel having one capillary, a reaction vessel employed in the present invention may have two or more capillaries.

A reaction vessel having a capillary-shaped internal structure is exemplified in the example described above, the structure is not limited to a capillary-shape, and may be any form as long as it allows a desired reaction to be conducted therein.

A capillary array employed in the invention can be modified for example as described below. FIG. 1(c) is another sectional view at a line 1B-1B in FIG. 1(a). As indicated in the sectional view shown in FIG. 1(c), a heater 6 and a temperature sensor 7 may be provided below the reaction zone 2. In such a case, the heater 6 may be provided for example below a polyimide thin film 5 having the probe nucleic acid 3 immobilized thereon, and the temperature sensor 7 may be provided below the heater 6. Such a device can be produced by any means known per se. The positions and the alignment patterns of the heater and the sensor contained in such a device may vary as desired. The heater and the sensor are not necessarily provided as integrated with the substrate.

For a capillary array described in this Example, see USP Unexamined Application publication No. 20020013457 (Akira Suyama). For other capillary arrays, see USP 6,143,152 (Simpson et al.).

A capillary array is constructed so that it has a constant sectional area throughout the entire length as described in USP Unexamined Application Publication No. 20020013457 (Akira Suyama). As a result, the liquid volume is uniform everywhere in the channel, thus allowing an equal reaction condition. For the same reason, the liquid is charged into and discharged from the capillary array in a quantitatively adjusted manner without using a particular controlling means, thus achieving a smooth liquid handling without any pulsating flow.

### Example 2

### (1) Reaction vessel

An example of a method for determining the type of a base at a target site in a target nucleic acid is shown below, wherein a reaction vessel similar to the reaction vessel in Example 1 is used except for having four capillaries, i.e., capillary 1, capillary 2, capillary 3 and capillary 4.

On a glass plate as a first substrate, four grooves each of 3 to 4 cm in length, 1 mm in width and 0.1 to 0.2 mm in height were formed, and through-holes were formed at the both ends of each groove. As a second substrate, a streptavidin-coated slide.(Greiner Japan) was employed. On this streptavidin-coated slide, an RV probe (ggaaacagctatgaccatg; SEQ ID No: 1) labeled with biotin at the 5' end was immobilized using a depositing machine. The immobilization was conducted so that, upon binding the first and the second substrates, the RV probes were provided in the respective positions corresponding to the four grooves. This immobilization utilized biotin-avidin reaction. As a result of such a binding of the first and second substrates, the four grooves served to form four capillaries, namely, capillary 1, capillary 2, capillary 3 and capillary 4. As a result, the RV probe nucleic acids were provided in the four capillaries thus formed. Using the reaction vessel thus produced, the following experiment was conducted.

### (2) Gene variation analysis

An example of a method for analyzing a gene variation is described below. In the experiment, using a sample target nucleic acid whose fourth base "N" is unknown (tgcNcatggtcatagctgtttcc), whether the "N" is adenine or guanine is determined.

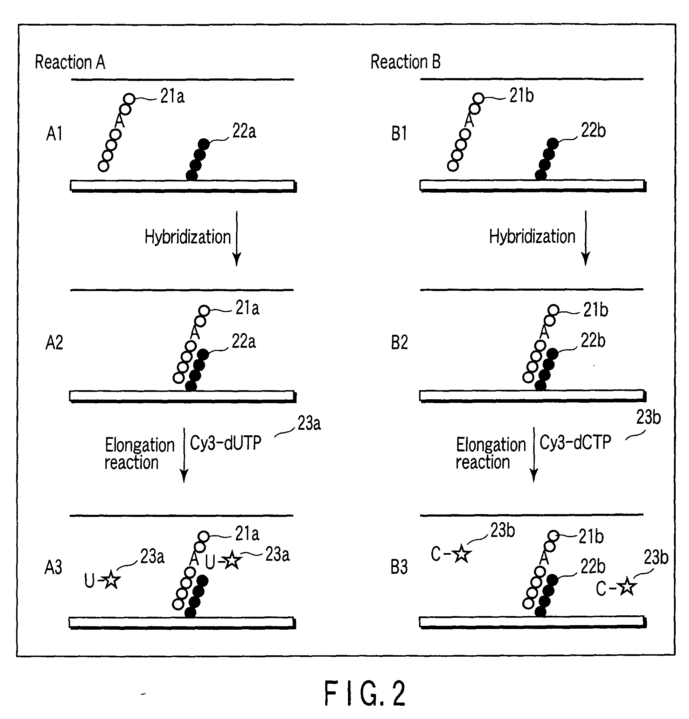

The capillary 1 and capillary 2 were charged with RV comp target (tgcacatggtcatagctgtttcc; SEQ ID No: 2) as a first target nucleic acid at 100 nM (in 1 × SSC solution). Similarly, the capillary 3 and capillary 4 were charged with RV comp target (tgcgcatggtcatagctgtttcc; SEQ ID No: 3) as a second target nucleic acid at 100 nM (in 1 × SSC solution). They were hybridized with the RV probes of SEQ ID No: 1 for 1 hour at 37°C. After the hybridization, any unreacted target nucleic acid was removed from the inside of the capillaries by washing thoroughly with the 1 × SSC solution (composition: 0.15 M NaCl, 0.015 M sodium citrate, pH 7.0). Then, 0.02 ml of an elongation reaction solution A [10 mM Tris-HCl (pH 7.5), 7 mM MgCl₂, 0.1 mM DTT, Klenow Fragment (DNA polymerase I, Large Fragment; TOYOBO)] at 0.4 units/µL and 10 µM Cy3-dUTP were added to the capillaries 1 and 3 (FIG. 2, Reaction A), and an elongation reaction solution C [10 mM Tris-HCl (pH 7.5), 7 mM MgCl₂, 0.1 mM DTT, Klenow Fragment (DNA polymerase I, Large Fragment; TOYOBO)] at 0.4 units/µL and 10 µM Cy3-dCTP were added to the capillaries 2 and 4 (FIG. 2, Reaction B), and they were reacted for 1 to 2 hours at 37°C.

After the reaction, any unreacted material was removed by washing thoroughly with 0.1 × SSC, and then the fluorescent intensity was measured. The results are shown in Table 1. Table 1 indicates a relative value regarding the highest fluorescent intensity as 1.

**Table 1**

| | Fluorescent intensity (relative value) | Target nucleic acid N | dNTP |
|---|---|---|---|
| Capillary 1 | 1.000 | a | UTP |
| Capillary 2 | 0.012 | a | CTP |
| Capillary 3 | 0.017 | g | UTP |
| Capillary 4 | 0.952 | g | CTP |

As indicated by the results shown in Table 1, the capillaries 1 and 4 exhibited intense fluorescence. Based on these results, the elongation reaction was taken place in the capillaries 1 and 4, while no elongation reaction was taken place in the capillaries 2 and 3. Accordingly, the base on the target site in the target nucleic acid added to the capillary 1 is judged to be adenine, while the base on the target site in the target nucleic acid added to the capillary 4 is judged to be guanine.

Thus, the base represented by "N" in the sample target was determined based on the type of the nucleotide substrate contained in the elongation reaction solution added and on the presence or absence of the elongation reaction.

In the case described above, the target nucleic acids having known sequences, i.e., the RV comp target (tgcgcatggtcatagctgtttcc; SEQ ID No: 3) and the RV comp target (tgcacatggtcatagctgtttcc; SEQ ID No: 2), were employed for convenience to illustrate a method according to an embodiment of the invention. Also in the case of a sequence whose base at the target site is not known, the base can similarly be determined. If a gene mutation was detected according to such an embodiment, then a polymorphic gene type can be determined.

An event resulting from such a reaction is shown schematically in FIG. 2. In this reaction, as shown in (A1) and (B1), target nucleic acids 21a and 21b were used as nucleic acid samples containing the sequences complementary to selection sequences, and probe nucleic acids 22a and 22b were used as probe nucleic acids containing selection sequences. The base corresponding to the target site "N" in the target nucleic acid was "A". The reaction A is a reaction generated in the capillary 1, while the reaction B is a reaction generated in the capillary 2. In the reaction A, a fluorescent labeled base "U" 23a (i.e., Cy3-dUTP) was employed as a labeled substrate nucleic acid (A3). In the reaction B, a fluorescent labeled base "C" 23b (i.e., Cy3-dCTP) was employed as a labeled substrate nucleic acid (B3). As used herein, each abbreviation denotes as follows: "A" is adenine, "U" is uracil, "T" is thymine, "G" is guanine and "C" is cytosine. The base exemplified herein is exemplified just for convenience of explanation, and is not intended to pose any limitation.

An event occurring in the reaction A is first described.
(A1) To a capillary on which a probe nucleic acid 22a is immobilized, a sample containing a target nucleic acid 21a is added.
(A2) The condition inside of the capillary is made appropriate for allowing the hybridization to occur. As a result, the target nucleic acid 21a and the probe nucleic acid 22a are hybridized.
(A3) Then, the Cy3-labeled dUTP 23a is added as a substrate, and the inside of the capillary is made appropriate for allowing an elongation reaction to occur. As a result, the dUTP 23a is incorporated into the probe nucleic acid 22a, thus effecting the elongation of the probe nucleic acid 22a.

Next, an event occurring in the reaction B is described.
(B1) To a capillary on which a probe nucleic acid 22b is immobilized, a sample containing a target nucleic acid 21b is added.
(B2) The condition inside of the capillary is made appropriate for allowing the hybridization to occur. As a result, the target nucleic acid 21b and the probe nucleic acid 22b are hybridized.
(B3) Then, the Cy3-labeled dCTP 23b is added as a substrate, and the inside of the capillary is made appropriate for allowing an elongation reaction to occur. As a result, the dCTP 23b is not hybridized with the base in the position of "N" in the target nucleic acid 21b and no elongation of the probe nucleic acid 22b occurs, since the dCTP 23b has no complementarity with the base in the position of "N".

In these reactions, each of the target nucleic acids 21a and 21b contains the sequence complementary to the selection sequence and also contains, on the 5' side of the complementary sequence, a sequence different from the selection sequence or complementary sequence. On the other hand, probe sequences 22a and 22b have selection sequences. Subsequently the 3' ends of the probe sequences 22a and 22b are elongated using as a template the 5' side-sequence of the complementary sequence of the target nucleic acid hybridized therewith.

Using the reaction vessel having a reaction zone in the form of a capillary, the amounts of the sample and the reagents become less advantageously. However, the reaction vessel employed in the invention is not limited to one having a reaction zone in the form of a capillary. In the case described above, a device equipped with a single capillary in a single reaction vessel is exemplified, but a reaction vessel is not limited to the device, and may be one having a reaction zone whose internal structure possesses a plurality of capillaries in a single reaction vessel.

While a single probe nucleic acid is indicated in the schematic view mentioned above for convenience of explanation, the invention is not limited to the example. Also the sequence of the probe nucleic acid is not limited to the sequence described above, and any sequence can be selected as desired. Probe nucleic acids having a plurality of different sequences respectively may also be employed on a single substrate, or a plurality of probe nucleic acids of an identical type may be employed.

According to the embodiment of the invention described above, it is readily possible to label a probe nucleic acid simultaneously with the determination of a base at a target site contained in a target nucleic acid.

### Example 3

Using a reaction vessel described in Example 2, Example 3 was conducted similarly.

In this example, the reaction was conducted similarly to Example 2, except for using a solution [10 mM Tris-HCl (pH 7.5), 7 mM MgCl₂, 0.1 mM DTT, Klenow Fragment (DNA polymerase I, Large Fragment; TOYOBO) at 0.4 units/µL, 10 µM Cy3-ddUTP] in place of an elongation reaction solution A employed in Example 2 and using a solution [10 mM Tris-HCl (pH 7.5), 7 mM MgCl₂, 0.1 mM DTT, Klenow Fragment (DNA polymerase I, Large Fragment; TOYOBO) at 0.4 units/µL, 10 µM Cy3-ddCTP] in place of an elongation reaction solution C.

The results of this reaction are shown in Table 2. Table 2 indicates a relative value regarding the highest fluorescent intensity as 1.

**Table 2**

| | Fluorescent intensity (relative value) | Target nucleic acid N | dNTP |
|---|---|---|---|
| Capillary 1 | 0.991 | A | UTP |
| Capillary 2 | 0.022 | A | CTP |
| Capillary 3 | 0.011 | G | UTP |
| Capillary 4 | 1.000 | G | CTP |

As indicated by the results shown in Table 2, the capillaries 1 and 4 exhibited intense fluorescence. Based on these results, the elongation reaction was taken place in the capillaries 1 and 4, while no elongation reaction was taken place in the capillaries 2 and 3. Accordingly, the base on the target site in the target nucleic acid added to the capillary 1 is judged to be adenine, while the base on the target site in the target nucleic acid added to the capillary 4 is judged to be guanine.

Thus, the base represented by "N" in the sample target was determined based on the type of the nucleotide substrate contained in the elongation reaction solution added and on the presence or absence of the elongation reaction.

In the case described above, the target nucleic acids having known sequences, i.e., the RV comp target (tgcgcatggtcatagctgtttcc; SEQ ID No: 3) and the RV comp target (tgcacatggtcatagctgtttcc; SEQ ID No: 2), were employed for convenience to illustrate a method according to an embodiment of the invention. Also in the case of a sequence whose base at the target site is not known, the base can similarly be determined. If a gene mutation was detected according to such an embodiment, then a polymorphic gene type can be determined.

### Example 4

Another example of a method for analyzing a gene variation is described below. In this example, using a sample target nucleic acid whose fourth base "N" is unknown (tgcNcatggtcatagctgtttcc), whether the "N" is adenine, guanine, cytosine or thymine is determined.

Example 4 was conducted using the same reaction vessel as that described in Example 1. The reaction vessel employed was produced as described below.

On a first substrate, one groove of 3 to 4 cm in length, 1 mm in width and 0.1 to 0.2 mm in height was formed on a glass plate, and through-holes were formed at the both ends of this groove. As a second substrate, a streptavidin-coated slide (Greiner Japan) was employed. On this streptavidin-coated slide, an RV probe (ggaaacagctatgaccatg; SEQ ID No: 1) labeled with biotin at the 5' end was immobilized using a depositing machine. The immobilization was conducted so that, upon binding the first and the second substrates, the RV probes were provided in the position corresponding to the groove.

As a result of such a binding of the first and second substrates, the groove served to form a single capillary.

The capillary was charged with RV comp target (tgcacatggtcatagctgtttcc; SEQ ID No: 2) as a target nucleic acid at 100 nM (in 1 × SSC solution). It was hybridized with the RV probe of SEQ ID No: 1 for 1 hour at 37°C. After the hybridization, any unreacted target nucleic acid was removed from the inside of the capillary by washing thoroughly with the 1 × SSC solution (composition: 0.15 M NaCl, 0.015 M sodium citrate, pH 7.0).

Then, 0.02 ml of an elongation reaction solution A [10 mM Tris-HCl (pH 7.5), 7 mM MgCl₂, 0.1 mM DTT, Klenow Fragment (DNA polymerase I, Large Fragment; TOYOBO)] at 0.4 units/µL, as well as 10 µM 6-FAM-dUTP, 10 µM HEX-dATP, 10 µM TAMRA-dCTP and 10 µM ROX-dGTP were added to the capillary, and they were reacted for 1 to 2 hours at 37°C.

After the reaction, any unreacted material was removed by washing thoroughly with 0.1 × SSC, and then the fluorescent intensity was measured. The fluorescent intensity measurement was conducted using as an excitation light an argon laser emitting an intense laser beam at 488 nm and 514 nm, and the fluorescent intensity was measured at a wavelength corresponding to each fluorescent substance employed as a marker substance. The wavelengths of the fluorescent substances employed were 513 nm for 6-FAM, 560 nm for HEX, 580 nm for TAMRA and 610 nm for ROX.

By using these four types of the fluorescent substances, the base at the target site can be determined efficiently within a single capillary.

### Example 5

Example 5 was conducted similarly to the Example 4 using the reaction vessel described in Example 4. Similarly to the method described in Example 4, a target nucleic acid was hybridized with a probe nucleic acid. Then, instead of the elongation reaction solution employed in Example 4, another elongation reaction solution [10 mM Tris-HCl (pH 7.5), 7 mM MgCl₂, 0.1 mM DTT, Klenow Fragment (DNA polymerase I, Large Fragment; TOYOBO)] at 0.4 units/µL, 10 µM dATP, 10 µM dCTP, either 0 µM dGTP or 10 µM ddGTP, and 10 µM Cy3-dUTP] was employed, and otherwise similarly to Example 4 the elongation reaction was conducted.

As a result of this reaction, the RV target was elongated from its 3' end to Cy3-U when 0 µM dGTP was employed. Thus, no further "G", "C" or "A" was elongated. When using 10 µM ddGTP, the RV target was elongated from its 3' end to "G". Thus, no further "C" or "A" was elongated.

Thus, the presence or absence of the variation can be detected.

### Example 6

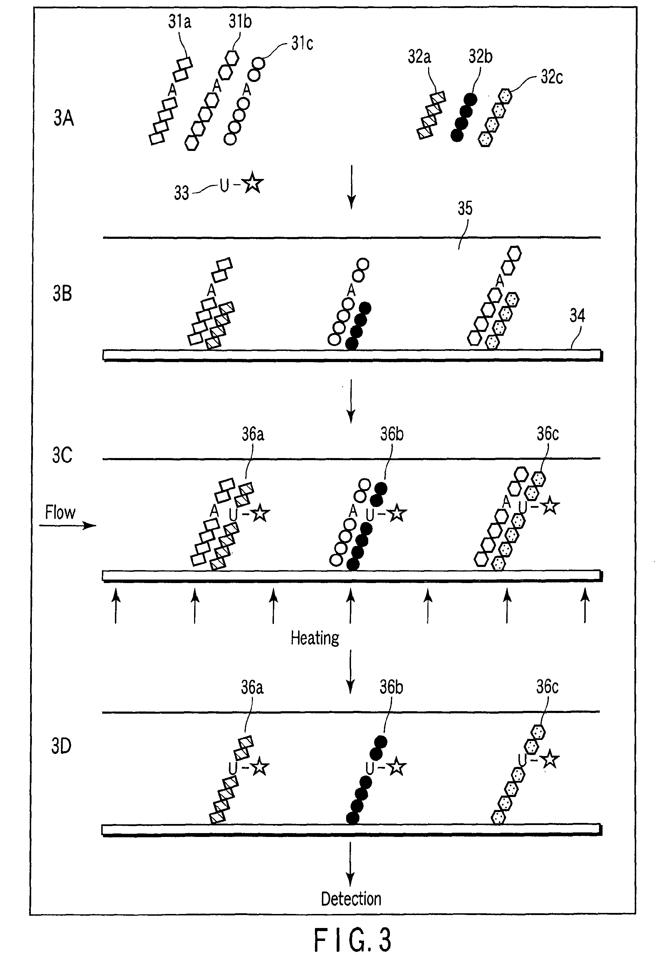

Example 6 is discussed using FIG. 3 as a schematic view. In this Example, a reaction vessel 30 is employed which is made similarly to Example 5 except for immobilizing a first, second and third probe nucleic acids 3a, 3b and 3c having respective different selection sequences. As shown in 3A, the base at the target site contained in each of the target nucleic acids 31a to 31c was indicated as "A" for convenience.

According to the method described in Example 5, the target nucleic acids 31a to 31c are first hybridized with the probe nucleic acids 32a to 32c as shown in 3B, and then subjected to an elongation reaction as shown in 3C. Then, in 3C, the temperature of a reaction zone 35 is elevated for example to about 95°C to dissociate the target nucleic acid 31 from an elongated probe nucleic acid 36, and then a buffer solution such as 0.1 × SSC was injected into the capillary having the reaction zone 35 and discharged from the capillary to let the inner solution flow. As a result, the dissociated target nucleic acid is removed whereby obtaining elongated and labeled probe nucleic acids 36a, 36b and 36c each as a single strand as shown in 3D.

While a heat treatment was conducted as a means for dissociating the double-stranded nucleic acid in the embodiment described above, the means for dissociating the double-stranded nucleic acid is not limited to this treatment in the invention. In other words, any method known per se which is used generally for dissociating a double-stranded nucleic acid into single strands may be employed. For example, an alkaline solution, urea or formamide may also be employed.

Also in the case where the target nucleic acid is an RNA and the probe nucleic acid is a DNA, the dissociation into single strands may be conducted using an enzyme degrading the RNA such as an RNAase H.

While a case of three types of the probe nucleic acids and the target nucleic acids was exemplified above, more or less number of the types of the probe nucleic acids and/or target nucleic acids may be employed, and one or more nucleic acids of various types may also be employed.

In this case, the fluorescent intensity was measured after dissociating a double-stranded nucleic acid into single strands, but the double strand may directly be subjected to the fluorescent measurement. Similarly in other cases disclosed in the specification, the fluorescent measurement may be conducted after dissociation into single strands.

According to an embodiment of the invention, a part of the method described in Examples 1 to 6 described above may be combined with each other as desired, or a part may be modified as desired.

According to a further aspect of the invention, a single-stranded labeled probe nucleic acid obtained by the embodiment of the invention of Example 6 may be provided as a further embodiment of the invention.

For example, an elongated and labeled probe nucleic acid according to an embodiment of the invention is converted into single strands, which can be utilized as a labeled probe nucleic acid in a nuclease protection assay as discussed in Example 7 described below.

In a conventional method, a highly sophisticated technology is required for labeling an intermediate site of a probe nucleic acid. Also when a probe nucleic acid whose intermediate site is labeled is prepared by a specialty company, an enormous time and expense are required. On the contrary, according to an embodiment of the invention, any desired intermediate position of a probe nucleic acid can readily be labeled, since the elongation reaction of a nucleotide can be controlled. Accordingly, a probe nucleic acid to be utilized in a nuclease protection assay can be prepared efficiently within a short period.

### Example 7

### Utilization in nuclease protection assay

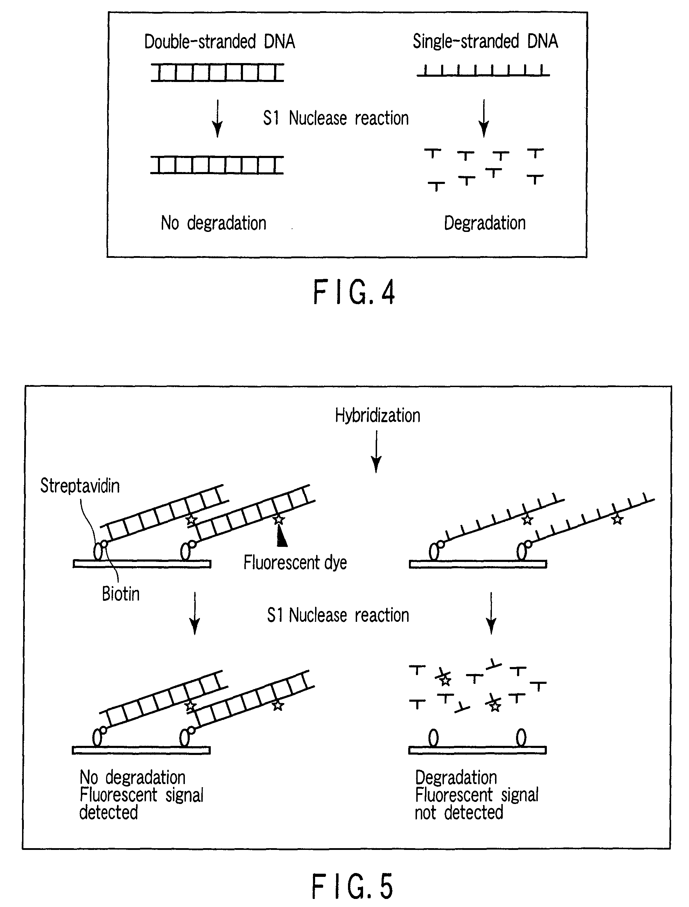

An S1 nuclease, which is a nucleic acid degradation enzyme (nuclease), is a single strand-specific endonuclease, and degrades both of a DNA and an RNA into an acid-soluble 5'-P nucleotide, and finally 90% or more of the entire amount is degraded into a 5'-P nucleotide. An S1 nuclease also acts on a single strand part of a double strand to effect degradation. This enzyme is employed frequently for removing single strand parts of DNA-DNA and DNA-RNA hybrids. An exonuclease I is also a single strand-specific exonuclease, and effects a hydrolysis of a single-stranded DNA in order from the 3' end to give a 5'-P nucleotide. These enzymes are employed for removing primers after a PCR (FIG. 4).

A nuclease protection assay is an analysis method in which an immobilized probe nucleic acid is labeled in advance, hybridized with a sample, and then reacted with a single strand-specific nuclease. For example, a double-stranded DNA hybridized with a target nucleic acid is protected from degradation by the nuclease whereby its label remains, while an unreacted probe nucleic acid (such as a single-stranded DNA) is degraded by the nuclease whereby its label is released. Accordingly, the level of the label on the protected probe nucleic acid is measured by detecting the signal from the marker substance contained in the probe nucleic acid, whereby determining the gene expression frequency (FIG. 5).

Such a nuclease protection assay can also be utilized as a part of the invention for analyzing a variation. For example, a single-stranded labeled probe nucleic acid obtained by the method described in Example 6 may be used to analyze a nucleic acid by a nuclease protection assay. Alternatively, a double-stranded labeled probe obtained by the method described in Examples 2 to 5 is converted into single strand by a means described in Example 6, and a single-stranded labeled probe nucleic acid thus obtained may be used. A single-stranded labeled probe thus obtained may be utilized as being immobilized on a substrate, in a nuclease protection assay, or may be utilized after release and recovery from the substrate. Furthermore, the use can be made after the recovery followed by purification.

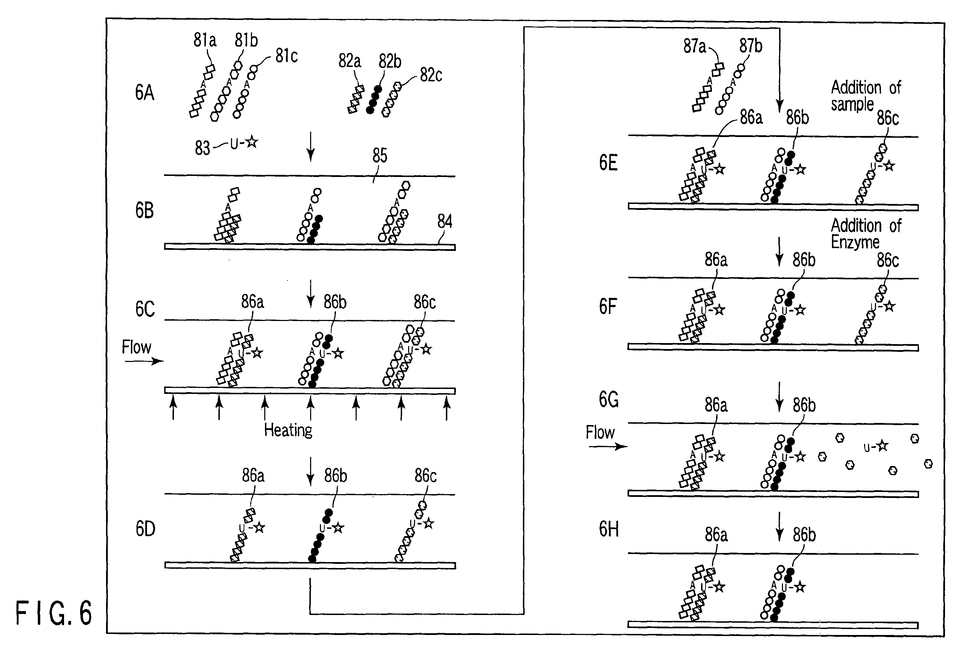

A further embodiment of the invention is discussed referring to a schematic view of FIG. 6. First, the stages before the detection of a fluorescent intensity, i.e., a procedure from 6A to 6D in FIG. 6 is conducted similarly to the method described in Example 6. As a result, a single-stranded labeled probe nucleic acid for a nuclease protection assay can be obtained (6D).

Then, a sample to be analyzed for the nucleic acid sequence is added and reacted with the probe under a condition enabling a hybridization reaction. As shown in 6E, when the target nucleic acid to be detected is present in the sample, then the hybridization occurs. In other words, the target nucleic acids 87a and 87b containing the target are hybridized with the labeled probe nucleic acids 86a and 86b, respectively. Thereafter, as shown in 6F, a single strand-specific endonuclease is added, and thereby the hybrids are subjected to a reaction under an appropriate condition. As a result, the labeled target nucleic acid 86c which did not undergo the hybridization is degraded (6G). Subsequently, the liquid in the reaction zone 85 is let flow to remove the degraded nucleic acid (6G) and then the fluorescent intensity is detected (6H).

The details with regard to the condition of a nucleotide protection assay employed by this embodiment may be determined appropriately by those skilled in the art.

In the embodiment described above, the processes from the probe nucleic acid elongation and the labeling through the nuclease protection assay are conducted continuously, but the invention is not limited to such an embodiment. An elongated and labeled probe nucleic acid may be prepared in advance as a single-stranded labeled probe nucleic acid by the elongation and the labeling of the probe nucleic acid, and may be used in the nuclease protection assay as desired.

The capillary-shaped reaction vessel is advantageous in that it is highly possible that the processes from dispensing and washing through measurement can be automated only by replacing respective solutions. Also in the case of a capillary-shaped reaction vessel, it is a further advantage that the solution contained can readily be replaced.

By using a labeled probe nucleic acid obtained as described above, a nuclease protection assay can gain an advantage described below. Specifically, since the probe nucleic acid can be labeled with a fluorescent substance in advance, the level of the immobilized probe nucleic acid can be known before hybridization. Further, it is possible to know the immobilized probe level or the deposited spot condition before a reaction. Accordingly, it is easy to control the accuracy of the immobilization of the probe nucleic acid. In addition, in the case of using an mRNA as a sample, it can be directly subjected to hybridization unlike a conventional method, and therefore it is possible to avoid any reduction of the efficiency resulting from the procedure such as cDNA preparation using a reverse transcriptase or labeling.

### Example 8

According to a further aspect of the invention, a single probe nucleic acid may be treated with a test sample containing a target nucleic acid a plurality of times repeatedly.

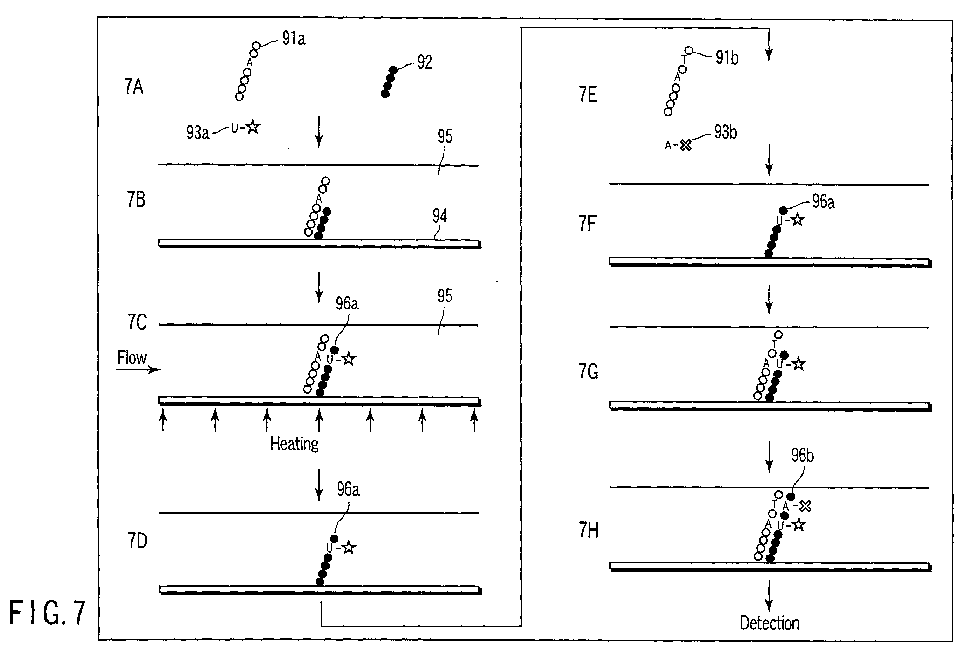

A case in which the treatment is repeated twice according to an embodiment of the invention using the same reaction vessel 1 as that described in Example 1 is discussed with referring to FIG. 7. FIG. 7 shows schematically the condition of each molecule in each step.

First, a probe nucleic acid 92 is immobilized on a bottom 94 of a reaction zone 95. Then, to the reaction zone 95, a first target nucleic acid 91a, a first fluorescent labeled substrate nucleic acid 93a (a labeled dUTP is shown in FIG. 7 as an example) and a non-labeled substrate nucleic acid (such as dCTP, dGTP and dATP; they are not shown in FIG. 7) are added under a condition enabling a hybridization reaction whereby effecting the hybridization (7B). Then, a polymerase is added to conduct an elongation reaction up to the base next to the first target site (indicated as "A" in FIG. 7) (7C).

Alternatively, at first, the probe nucleic acid 92 and the first target nucleic acid 91a may be hybridized with each other without adding the fluorescent labeled substrate nucleic acid 93a and the non-labeled substrate nucleic acid, and then the both substrate nucleic acids and the polymerase may be added to effect the elongation reaction.

In order to arrest the elongation reaction at a desired site, i.e. "the base next to the first target site", a dNTP having a base complementary to a further next base to "the base next to the first target site" is not added.

Subsequently, as shown in 7C, the temperature of the reaction zone 95 filled for example with a 0.1 × SSC solution is raised to 95°C to dissociate the target nucleic acid 91a from the elongated probe nucleic acid 96a. The dissociated nucleic acid 91a is removed by injecting a solution such as 0.1 × SSC into the capillary having the reaction zone 95 and discharging it from the capillary to let the inner solution flow. As a result, as shown in 7D, a elongated and labeled target nucleic acid 96a is obtained as a single strand.

Subsequently, as shown in 7E, a second target nucleic acid 91b, a second fluorescent labeled substrate nucleic acid 93b (a labeled dATP is shown in FIG. 7 as an example) and a non-labeled substrate nucleic acid (such as dCTP, dGTP and dUTP; they are not shown in FIG. 7) are added in the presence of the elongated and labeled nucleic acid 96a under a condition enabling a hybridization reaction whereby effecting the hybridization (7G). Then, a polymerase is added to conduct an elongation reaction up to the base next to the second target site (indicated as "T" in FIG. 7) (7H). Then, a further elongated and labeled second probe nucleic acid 96b is examined for the fluorescent intensities of the first fluorescent substance and the second fluorescent substance (7H).

Alternatively, at first, the probe nucleic acid 96a and the target nucleic acid 91b may be hybridized with each other without adding the fluorescent labeled substrate nucleic acid 93b and the non-labeled substrate nucleic acid, and thereafter the both substrate nucleic acids and the polymerase may be added to conduct the elongation reaction.

In this embodiment, the elongation reaction was effected up to the base next to the second target site, but a further elongation reaction may be conducted or the elongation reaction may be arrested at the second target site.

In this embodiment, the obtained probe nucleic acid contains a single base between the first target site and the second target site, but the invention is not limited to this embodiment. The base may not be located between the both target sites, or two or more bases may be located between the target sites.

As shown in 7H, the first fluorescent substance (indicated by star in FIG. 7) and the second fluorescent substance (indicated by X in FIG. 7) contained in the elongated and labeled probe nucleic acid 96b thus obtained are preferably distinguishable, and preferably these substances emit fluorescence at wavelengths different from each other. The wavelengths may not always be different depending on the selection of the detecting means, as in a case of the judgment based on the difference in the fluorescent intensity.

The elongated and labeled probe nucleic acid 96b thus obtained may be converted into a single strand and subjected to a further repeat of the method described above.

According to an embodiment of the invention, a highly efficient gene variation analysis method is provided.

According to an embodiment of the invention, it is possible to selectively label only a probe nucleic acid that is hybridized with a target nucleic acid containing a target site to be detected in a reaction vessel. Therefore, the invention makes it possible to analyze a gene variation efficiently, and at the same time the invention makes it possible to label a probe nucleic acid efficiently. Thereby, the production cost for the reaction vessel having the labeled probe is reduced.

According to an embodiment of the invention, a gene variation analysis can be conducted without need of a complicated procedure.

According to an embodiment of the invention, an entire processing from the labeling through the expression frequency analysis can be conducted in a single container all at once.

### 2. Gene expression analysis method

### 2.1 Outlines of gene expression analysis method

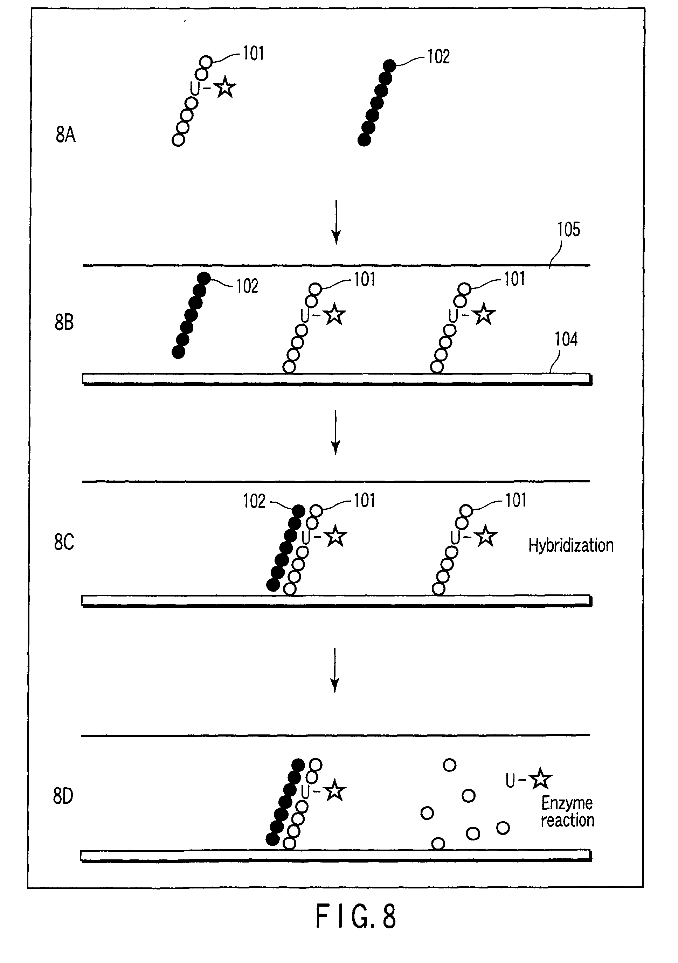

According to the invention, a method for detecting the presence of a target sequence in a test nucleic acid is provided. One embodiment of the method for detecting the presence of the target sequence in a test nucleic acid is explained with referring to FIG. 8.

First, a target nucleic acid 102 having a target sequence to be detected is selected. On the other hand, a probe nucleic acid 101 which has a sequence complementary to the target sequence described above and which also has a marker substance attached to a part of the bases of this sequence is provided (FIG. 8, 8A). Then, the probe nucleic acid 101 is immobilized on a bottom 104 of a reaction zone 105. To the reaction zone 105 on which the probe nucleic acid 101 is immobilized, a test nucleic acid is added (8B), and reacted under a condition enabling an appropriate hybridization (8C). Here if a target nucleic acid having the target sequence is present in the test nucleic acid, then the hybridization with the probe nucleic acid occurs and a double-stranded nucleic acid arises.

Subsequently, a single strand-specific nuclease is added to the reaction zone, and the reaction is conducted under a condition enabling an appropriate enzyme reaction (8D). As a result, any single-stranded nucleic acid remaining in this reaction zone, such as a probe nucleic acid or target nucleic acid still remaining as a single strand is degraded by this enzyme. After this enzyme reaction, the reaction zone is washed, and the signal from the marker substance contained in the double strands remaining there is detected, whereby detecting the presence of the target nucleic acid having the target sequence in the test nucleic acid.

Also in this procedure, by using as a test nucleic acid a sample containing a nucleic acid taken out from a subject and conducting the detection as described above, a method for analyzing the gene expression frequency in the subject is also provided.

In a nucleotide protection assay utilized in the invention, a step of allowing a liquid containing a nuclease to flow during the reaction of degradation by the nuclease may be included. As a result of this flowing, the nuclease exerts a degradation activity uniformly over the immobilized labeled probe nucleic acids corresponding to various types of the targets. Furthermore, by allowing a solution containing a nuclease to flow in one direction continuously or intermittently in the channel such as a capillary array, the degradation reaction and the removal of the degraded product can continuously be conducted. Thereby, the enzyme reaction can be maintained in the active condition without undergoing a reduction in the enzyme activity which occurs in a static condition. On the other hand, a nucleotide protection assay utilized in the invention may involve a step of determining the reaction level accurately, based on the actual immobilization amount, by measuring the amounts of the label before and after the reaction with a sample and comparing the both amounts of the label.

A single strand-specific nuclease employed in such a reaction is a nuclease which degrades a single-stranded nucleic acid selectively. Among those, an S1 nuclease degrades both of a DNA and an RNA into an acid-soluble 5'-P nucleotide, and finally 90% or more of the entire amount is degraded into a 5'-P nucleotide (FIG. 4). It also acts on a single strand part of a double strand to effect degradation. This enzyme is employed frequently for removing single strand parts of DNA-DNA and DNA-RNA hybrids. Further, an exonuclease I is a single strand-specific exonuclease, and it hydrolyzes a single-stranded DNA in order from the 3' end to give a 5'-P nucleotide. These enzymes are employed conventionally for removing primers after a PCR.

A conventional nuclease protection assay is an analysis method in which an immobilized probe nucleic acid is labeled in advance, hybridized with a sample, and then reacted with a single strand-specific nuclease. For example, a double-stranded DNA hybridized with a target nucleic acid is protected from degradation by the nuclease whereby its label remains, while an unreacted probe nucleic acid (such as a single-stranded DNA) is degraded by the nuclease whereby its label is released (FIG. 5). Accordingly, the amount of the marker on the protected probe nucleic acid is measured by detecting the signal from the marker substance contained in the probe nucleic acid, whereby determining the gene expression frequency. Such a conventional protection assay is described for example in PCT National Publication 2000-512499 (USP 5,770,370).

In contrast to a conventional method described in the publication mentioned above, the invention has the following advantage. According to an embodiment of the invention, an inventive method described above is conducted preferably in a reaction vessel having a capillary-shaped reaction zone capable of temperature controlling as detailed below. By using a reaction vessel having a capillary-shaped reaction zone and by conducting pre-operation before the reaction (for example, immobilization and labeling) in an identical capillary, all procedures and reactions can efficiently be accomplished. Also advantageously, a complicated procedure required conventionally becomes unnecessary, and the amount of a sample required becomes very small. An automation is also possible since all reaction can be conducted collectively in a small vessel. Also according to an embodiment of the invention, the condition of the immobilization of a probe before reaction can be monitored. Moreover, unlike an analysis on the basis of a competitive reaction as in a conventional DNA array, an embodiment of the invention enables a comparison between other samples, comparison between capillaries, and comparison between plates. In addition, a reduction in the efficiency attributable to a cDNA preparation using a reverse transcriptase and a labeling process can also be avoided.

As mentioned above, an expression analysis method according to the invention is conducted preferably in a reaction vessel 1 having a capillary-shaped reaction zone 2 as shown in FIG. 1.

As used herein, the term "subject" means any animal such as mammals including human, monkey, cattle, pig, sheep, goat, dog, cat, rabbit, rat and mouse, and reptiles, fishes and insects, as well as other living individuals capable of expressing genes, as well as a cell and a tissue obtained from any individuals.

As used herein, the term "test nucleic acid" is a nucleic acid serving as a object for the detection of the presence of a target sequence. A test nucleic acid may be any naturally occurring nucleic acid or may be an artificially synthesized nucleic acid. For example, in the case of analyzing a gene expression frequency, an mRNA expressed in a subject may generally be a test nucleic acid.

A step of obtaining a test nucleic acid from a subject can be conducted by a means known per se, and when the test nucleic acid is an mRNA, a commercially available kit may be used or an oligo dT column may be used. However, such a means is not limitative. The test nucleic acid may, after being extracted from a subject, be amplified by a method known per se and then subjected to a method according to an embodiment of the invention.

A "template nucleic acid" and a "seed probe nucleic acid" used for obtaining a labeled probe nucleic acid may be any nucleic acid. As used herein, the term "nucleic acid" means any of various naturally occurring DNAs and RNAs, as well as artificially synthesized nucleic acid analogues such as peptide nucleic acids, morpholino nucleic acids, methylsulfonate nucleic acids and S-oligo nucleic acids and the like, and its nucleotide sequence or the presence or absence of a modification may be selected as desired.

As used herein, the term "target sequence" means a nucleotide sequence to be detected. As used herein, the term "target nucleic acid" means a nucleic acid containing a target sequence that is a nucleotide sequence to be detected.

As used herein, the term "probe nucleic acid" means a nucleic acid for detecting a target sequence, and it contains as its part a nucleotide sequence complementary to the target sequence. As used herein, the term "labeled probe nucleic acid" means a nucleic acid in which a marker substance is bound to a base in the sequence of the probe nucleic acid. A probe nucleic acid according to an embodiment of the invention is immobilized on a substrate via its 5' end. The immobilization of the probe nucleic acid can be conducted by immobilizing a desired probe nucleic acid on any of known substrates.

As used herein, the term "substrate" according to an embodiment of the invention may be in any form allowing a hybridization reaction to be conducted there. For example, it may be a generally employed reaction vessel, including a reaction vessel having a reaction zone for conducting a reaction therein such as a capillary or well, or may be a plate or spherical substrate for conducting the reaction on its surface. Alternatively, the "substrate" may be a needle, strand, fiber, disc or porous filter. Because of an easy handling, a reaction vessel having a reaction zone in the form of a capillary is preferred.

In the case of a reaction vessel having a capillary-shaped reaction zone, the capillary-shaped reaction zone may be formed by a groove having a width and a thickness of about 10 to about 50 µm which is formed on a substrate such as a silicon board or a glass board. The capillary-shaped reaction zone provided in a single reaction vessel may be about 50 to about 100 capillaries (grooves) per cm, at an interval of about 50 µm, in a single reaction vessel. Each capillary preferably has a structure allowing the reaction temperature to be reached rapidly.

As used herein, the term "marker substance" means a substance capable of generating a detectable signal, for example, fluorescent substance, radioactive substance and chemiluminescent substance. A substance developing a color upon an enzyme reaction may also be employed. As in the case where a plurality of nucleic acids are detected as target sequences or in the case where a plurality of probe nucleic acids are employed on a single substrate, a plurality of distinguishable marker substances may simultaneously be employed if desired.

A single strand-specific nuclease employed in an inventive method may be an enzyme capable of degrading a single-stranded nucleic acid selectively. Such an enzyme may be any enzyme known per se, for example, S1 nuclease and exonuclease I.

### 2.2 Method for preparing labeled probe nucleic acid

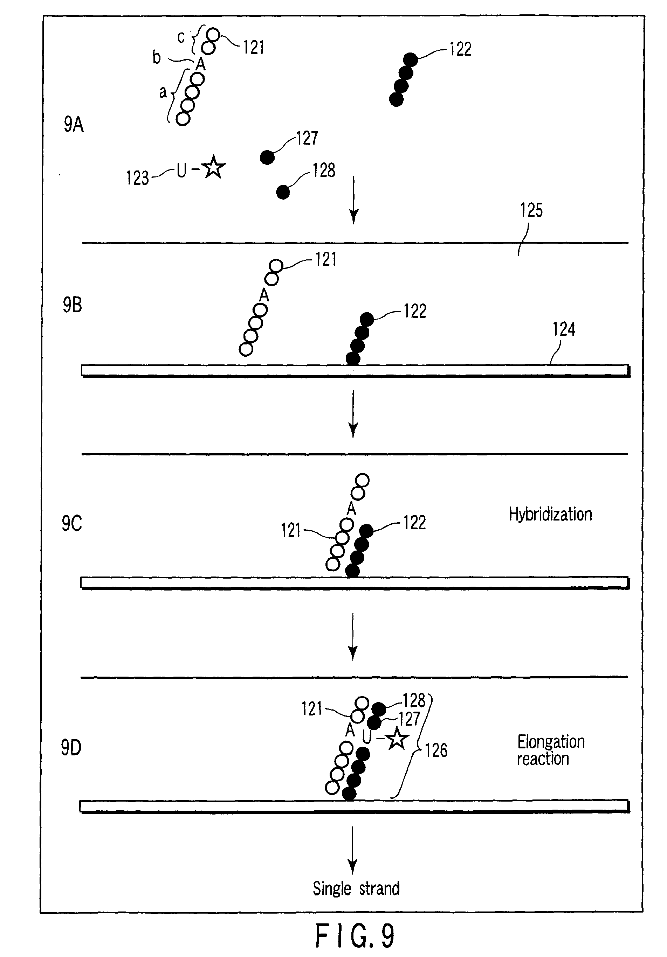

In the present invention, any means known per se employed for binding a marker substance to a desired site in a probe nucleic acid may be used in order to prepare a "labeled probe nucleic acid" employed in the invention. Further, a method for preparing a labeled probe nucleic acid described below may be used in order to prepare a "labeled probe nucleic acid" used in a gene expression analysis method described above. A method for preparing a "labeled probe nucleic acid" is explained below with referring to FIG. 9.

First, a seed probe nucleic acid 122 having a template capture sequence is immobilized on a bottom 124 of a reaction zone 125 via its 5' end. Then, a template nucleic acid 121 is added to the reaction zone 125 and reacted under a condition enabling an appropriate hybridization, for example a stringent condition. Here, the template nucleic acid 121 contains at its 3' side a sequence "a" complementary to the template capture sequence, a sequence "b" for labeling adjacent to the 5' side of the sequence "a", and a sequence "c" for elongating adjacent to the 5' side of the sequence "b" for labeling (FIG. 9, 9A). As a result of such a structure, the template nucleic acid 121 hybridizes with the seed probe nucleic acid 122. In FIG. 9, a case in which the sequence "b" for labeling is "A" (i.e., adenine) is indicated.

After the hybridization, the template nucleic acid 121 which was not used in the hybridization is removed.

Subsequently, to the reaction zone 125, a polymerase, a labeled deoxyribonucleoside triphosphate 123 containing a specific one base labeled with a marker substance generating a detectable signal, and non-labeled deoxyribonucleoside triphosphates 127 and 128 containing bases complementary to the sequence "c" for elongating are added, and the elongation reaction is conducted under a condition enabling an appropriate elongation reaction (9D). After completion of the elongation reaction, the nucleotide substrates which were not used in the elongation reaction are removed. Thereafter, the double strand obtained is denatured and the template nucleic acid 121 is removed. As a result, a labeled probe nucleic acid 126 is obtained. The labeled probe nucleic acid 126 thus obtained may be used as still being immobilized on the reaction zone 125, or may be used after recovery.

In the case described above, substances which were not subjected to the reaction were removed after the hybridization and the elongation reaction. However, such a removal may not always be necessary.

The length of a template nucleic acid employed here may vary depending on the length of an intended labeled probe nucleic acid. For example, the length of the template nucleic acid may be of 10 bases to 1000 bases, preferably 15 bases to 200 bases.

The length of a seed probe nucleic acid may be of 10 bases to 50 bases, preferably 15 bases to 30 bases. While adenine is employed as a base of a sequence for labeling in the case described above, it is not limitative, and may be any of cytosine, uracil, thymine and guanine.

A sequence for elongating is a sequence which determines the type and the length of a non-labeled nucleotide substrate which is incorporated subsequently after a labeled nucleotide substrate is incorporated into a seed probe. Accordingly, it can be selected by an operator as desired. However, it is not necessary always that the sequence for elongating exists. When the sequence for elongating does not exist, then a labeled nucleotide substrate exists at the end of the seed probe nucleic acid. The length of the sequence for elongating may be of 1 base to 50 bases, preferably 1 base to 10 bases.

According to the invention, the embodiments described in the following [1] to [6] are also provided preferably as inventions.
[1] A labeled probe nucleic acid obtained by the following steps:
   (1) a step of reacting a seed probe nucleic acid with a template nucleic acid containing a complementary sequence complementary to the seed probe nucleic acid, a sequence for labeling present on the 5' side of this complementary sequence and a sequence for elongating present on the 5' side of the sequence for labeling, under a condition for enabling a appropriate hybridization;
   (2) a step of elongating the seed probe nucleic acid, in the presence of a polymerase, a labeled nucleotide substrate which contains a base complementary to the base of the sequence for labeling and which is labeled with a marker substance, and a non-labeled nucleotide substrate having a base complementary to the base contained in the sequence for elongating; and
   (3) a step of dissociating the double strand obtained in the step (2) to obtain a labeled probe nucleic acid as a single strand.
[2] A labeled probe nucleic acid obtained by the following steps:
   (1) a step of reacting a first seed probe nucleic acid with a template nucleic acid containing a first complementary sequence complementary to the first seed probe nucleic acid, a first sequence for labeling present on the 5' side of this first complementary sequence and a first sequence for elongating present on the 5' side of the first sequence for labeling, under a condition for enabling a appropriate hybridization;
   (2) a step of elongating the first seed probe nucleic acid contained in a double strand obtained in the step (1), in the presence of a polymerase, a first labeled nucleotide substrate which contains a base complementary to the base of the first sequence for labeling and which is labeled with a marker substance, and a non-labeled nucleotide substrate having a base complementary to the base contained in the first sequence for elongating;
   (3) a step of dissociating the double strand obtained in the step (2) to obtain a second seed probe nucleic acid;
   (4) a step of reacting the second seed probe nucleic acid obtained in the step (3) with a template nucleic acid containing a second complementary sequence complementary to a part of the sequence on the 3' side of the second seed probe nucleic acid, a second sequence for labeling present on the 5' side of this second complementary sequence and a second sequence for elongating present on the 5' side of the second sequence for labeling, under a condition for enabling an appropriate hybridization reaction;
   (5) a step of elongating the second seed probe nucleic acid contained in a double strand obtained in the step (4), in the presence of a polymerase, a second labeled nucleotide substrate which contains a base complementary to the base of the second sequence for labeling and which is labeled with a marker substance, and a non-labeled nucleotide substrate having a base complementary to the base contained in the second sequence for elongating; and
   (6) a step of dissociating the double strand obtained in the step (5) to obtain a labeled probe nucleic acid as a single strand.
[3] A labeled probe nucleic acid according to the above-mentioned [2], obtained by repeating the steps (4) to (6) needed times using as a seed probe nucleic acid the labeled probe nucleic acid obtained in the step (6).
[4] A labeled probe nucleic acid according to any one of the above-mentioned [1] to [3] wherein the seed probe nucleic acid is immobilized on a substrate via its 5' end.
[5] A method for detecting the presence of a target sequence in a test nucleic acid using a labeled probe nucleic acid according to any one of the above-mentioned [1] to [4], wherein the method comprises:
   (1) a step of reacting the test nucleic acid with a labeled probe nucleic acid, which is immobilized onto a channel allowing the reaction to be conducted there and which has a marker substance attached thereto, under a condition for enabling an appropriate hybridization reaction;
   (2) a step of adding, after the step (1), a single-stranded specific nuclease to the channel to conduct a reaction under a condition for allowing an appropriate enzyme reaction to be achieved;
   (3) a step of removing, after the step (2), a degradation product of the enzyme reaction from the channel;
   (4) a step of detecting a signal from the marker substance contained in the double-stranded nucleic acid in the channel; and
   (5) a step of detecting the presence of the target sequence in the test nucleic acid based on a signal detected in the step (4).
[6] A method for detecting the presence of a target sequence in a test sample according to the above-mentioned [5], wherein the labeled probe nucleic acid to which a marker substance is bound and which is used in the step (1) described in the above-mentioned [5] is produced by the following steps:
   (i) a step of reacting a seed probe nucleic acid with a template nucleic acid containing a complementary sequence complementary to the seed probe nucleic acid, a sequence for labeling present on the 5' side of this complementary sequence and a sequence for elongating present on the 5' side of the sequence for labeling, under a condition for enabling an appropriate hybridization,;
   (ii) a step of elongating the seed probe nucleic acid, in the presence of a polymerase, a labeled nucleotide substrate which contains a base complementary to the base of the sequence for labeling and which is labeled with a marker substance, and a non-labeled nucleotide substrate having a base complementary to the base contained in the sequence for elongating; and
   (iii) a step of dissociating the double strand obtained in the step (ii) to obtain a labeled probe nucleic acid as a single strand.

### 2.3 Examples

A gene expression analysis method of the invention can be conducted by immobilizing a labeled probe nucleic acid containing a sequence complementary to a target sequence to be detected onto a reaction vessel described in Examples 1 and 2.

### Example 9: Nuclease protection assay 1

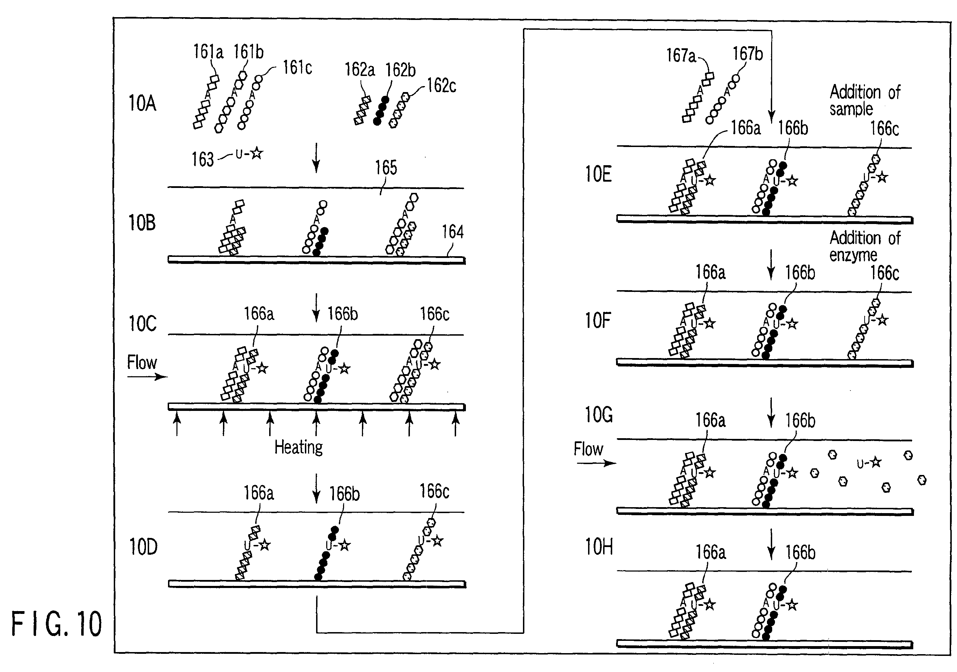

A method for detecting the presence of a target sequence in a test nucleic acid according to an embodiment of the invention is discussed below with referring to FIG. 10.

By means of any means known per se, a probe nucleic acid to which a marker substance is bound to a desired part is prepared. Such a labeled probe nucleic acid is immobilized on the bottom of a reaction vessel similar to the reaction vessel shown in Example 1 (10D).

For example, but not limited to, seed probe nucleic acids 162a to 162c are immobilized on a bottom 164 of a reaction zone 165 of a reaction vessel (10A); template nucleic acids 161a to 161c, a fluorescent labeled substrate nucleic acid 163 (a labeled dUTP is shown in FIG. 10 as an example) and a non-labeled substrate nucleic acid (such as dCTP, dGTP and dATP; they are not shown in FIG. 10) are added under a condition enabling a hybridization reaction whereby effecting the hybridization (10B); a polymerase is added to effect the elongation reaction up to the base desired, followed by heating or letting the inner solution flowing after heating to form a single strand (10C); and desired labeled probe nucleic acids 166a to 166c may be synthesized (10D). Although the fluorescent labeled and non-labeled substrate nucleic acids were allowed to be present upon hybridization, the fluorescent labeled and non-labeled substrate nucleic acids may be added simultaneously with the polymerase addition after completion of the hybridization.

Then, a sample to be subjected to an analysis of the nucleic acid sequence is added and reacted with a labeled probe nucleic acid under a condition enabling a hybridization reaction. As shown in 10E, when a target nucleic acid to be detected is present in the sample, then a hybridization reaction occurs. In the case exemplified here, target nucleic acids 167a and 167b containing the target sequences are hybridized with and probe nucleic acids 166a and 166b, respectively (10E).

Subsequently, a single strand-specific nuclease is added and reacted with a single stranded probe nucleic acid under an appropriate condition (10F).
As a result, a single-stranded probe nucleic acid 166c undergoing no hybridization is degraded (10G). Then, the content of the reaction zone 165 is let flow to remove the degraded nucleic acid (10G). Thereafter, the reaction zone 165 is subjected to the detection of the fluorescent intensity (10H).

The details with regard to the condition of a nucleotide protection assay employed by this embodiment may be determined appropriately by those skilled in the art.

In the embodiment described above, the processes from the seed probe nucleic acid elongation and the labeling through the nuclease protection assay are conducted continuously, but the invention is not limited to such an embodiment. A further elongated and labeled probe nucleic acid, which is obtained by the elongation and the labeling of the seed probe nucleic acid, may be prepared in advance as a single-stranded labeled probe nucleic acid for a nuclease protection assay and may be used in the nuclease protection assay at a desired time point. Alternatively, a single-stranded labeled probe nucleic acid is labeled at a desired position by a method known per se and may be used in the nuclease protection assay.

By using a labeled probe nucleic acid according to an embodiment of the invention, a nuclease protection assay can be performed with an advantage described below. Specifically, since the probe nucleic acid can be labeled with a fluorescent substance in advance, the level of the immobilized probe nucleic acid can be known before hybridization. Thus, it is possible to know the immobilized probe level or the deposited spot condition before a reaction. Accordingly, it is easy to control the accuracy of the immobilization of the probe nucleic acid. In addition, in the case of using an mRNA as a sample, it can be directly subjected to hybridization unlike a conventional method, and therefore it is possible to avoid any reduction of the efficiency resulting from the procedure such as cDNA preparation using a reverse transcriptase or labeling.

The capillary-shaped reaction vessel is advantageous in that it is highly possible that the processes from dispensing and washing through measurement can be automated only by replacing respective solutions. Also in the case of a capillary-shaped reaction vessel, it is a further advantage that the solution contained can readily be replaced.

### Example 10: Nuclease protection assay 2

First, a reaction vessel was produced by a method similar to that in Example 1. On a first substrate, a groove of 3 to 4 cm in length, 1 mm in width and 0.1 to 0.2 mm in height was formed on a glass plate, and through-holes were formed at the both ends of this groove. As a second substrate, a streptavidin-coated slide (Greiner Japan) was employed.

On this streptavidin-coated slide (manufactured by Greiner Japan), a PRH-HPRT 1M.BD whose 5' end is labeled with biotin and whose 10th base "T" from the 3' end is labeled with Cy3 (biotin-GGGGGCTATAAATTCTTTGC(T-Cy3)GACCTGCTG (SEQ ID No: 4)) was spotted at 10 nM using a depositing machine. As a result, a biotin-avidin reaction served to immobilize the probe nucleic acid. Thereafter, the first substrate and the second substrate were bound to each other.

Into a capillary formed as a groove in the reaction vessel, 10 nM of PRH-HRRT1RV.0 (CAGCAGGTCAGCAAAGAATTTATAGCCCCC (SEQ ID No: 5)) was added as a target nucleic acid. At this time, a 1 × SSC solution (0.15 M NaCl, 0.015 M sodium citrate) was employed as a solvent. A hybridization reaction was conducted for 1 hour at 42°C. After the hybridization, an unreacted target nucleic acid was removed from this capillary by washing thoroughly with a 1 × SSC solution. Then, an S1 nuclease solution (in buffer of 30 mM sodium acetate (pH 4.6), 280 mM NaCl and 1 mM ZnSO₄) was added into the capillary, and reacted for 1 hour at 37°C. After the reaction, any unreacted substances were removed by washing thoroughly with a 0.1 × SSC, and the fluorescent intensity was measured. Table 3 shows the fluorescent intensities relatively in the presence of 10 nM of the PRH-HPRT1RV.0 target nucleic acid and in the absence of this target nucleic acid.

**Table 3**

| PRH-HPRT1RV.0 target | Fluorescent intensity (relative value) |
|---|---|
| 10 nM | 1.000 |
| 0 nM | 0.012 |
| PRH-HPRT1M.BD probe 10 nM | |

The fluorescent intensity shown in Table 3 is a relative value regarding the highest fluorescent intensity as 1. As evident from the table, a method according to this embodiment enables a quantitative detection of the target nucleic acid.

According to an embodiment of the invention, only the probe nucleic acid hybridizing with the target nucleic acid having a target sequence can allow a signal from its marker substance to be detected selectively in the reaction vessel. Accordingly, the invention makes it possible to analyze a gene expression frequency efficiently.

According to an embodiment of the invention, a gene expression frequency can be analyzed without requiring complicated procedures. Also, according to an embodiment of the invention, all processes from the labeling through the expression frequency analysis can be conducted collectively in a single vessel.

The analysis of the gene expression frequency according to the invention is not a competitive reaction such as in a conventional analysis method, and therefore it enables a comparison between the reaction vessels in which other samples were subjected to detection. Thus, the invention makes it possible a comparison among a plurality of reaction vessels (for example, between capillaries, between plates).

In addition, since it is possible to know the immobilization state of the probe nucleic acid, it is possible to know the immobilized probe level or the deposited spot condition before a reaction. Accordingly, it is easy to control the accuracy of the immobilization of the probe.

### Example 11: Preparation of labeled probe 1

The following experiment was conducted using a reaction vessel shown in Example 1 described above.

RV comp target (tgcacatggtcatagctgtttcc; SEQ ID No: 2) complementary to the RV nucleic acid was added to a capillary as a template nucleic acid at 100 nM (in 1 × SSC solution), and hybridized with the RV nucleic acid for 1 hour at 37°C. After the hybridization, any unreacted template nucleic acid was removed from the inside of the capillary by washing thoroughly with a 1 × SSC solution (composition: 0.15 M NaCl, 0.015 M sodium citrate, pH 7.0). Then, 0.02 ml of an elongation reaction solution A [10 mM Tris-HCl (pH 7.5), 7 mM MgCl₂, 0.1 mM DTT, Klenow Fragment (DNA polymerase I, Large Fragment; TOYOBO) at 0.4 units/µL, 10 µM dATP, 10 µM dCTP, 10 µM dGTP and 10 µM Cy3-dUTP] was added to the capillary, and they were reacted for 1 to 2 hours at 37°C. After the reaction, any unreacted material was removed by washing thoroughly with 0.1 × SSC, and then the fluorescent intensity was measured (Table 4).

Table 4 shows the fluorescent intensities observed in the presence and absence of Klenow Fragment in the case of 10 nM or 1 nM of a deposition level of the seed probe nucleic acid on the substrate. A value in the table is a relative value regarding the highest fluorescent intensity as 1.

**Table 4**

| | | |
|---|---|---|
| Concentration of spot-deposited target | 10 nM | 1 nM |
| Addition of Klenow Fragment | 1.000 | 0.321 |
| No addition of Klenow Fragment | 0.017 | 0.012 |

As shown in Table 4, the absence of Klenow Fragment during the elongation reaction resulted in an extremely low relative value. Accordingly, it was proven that the binding of the labeled nucleic acid to the seed probe nucleic acid is not non-specific but is a result of the elongation by the polymerase. The relative fluorescent intensity was also increased in a manner dependent on the increase in the deposition level of the seed probe nucleic acid.

The event occurring from such a reaction is shown schematically in FIG. 9. In this reaction, as shown in 9A, an RV nucleic acid (ggaaacagctatgaccatg; SEQ ID No: 1) labeled with biotin at the 5' end was employed as a template nucleic acid 121 and an RV comp target (tgcacatggtcatagctgtttcc; SEQ ID No: 2) was employed as a probe nucleic acid 122. "A" was used as a base present in a sequence "b" for labeling, and a fluorescent labeled base "U" 23 was used as the labeled substrate nucleic acid containing the base complementary to the base "A" (9A). As used herein, each abbreviation denotes as follows: "A" is adenine, "U" is uracil, "T" is thymine, "G" is guanine and "C" is cytosine. Also as used herein, any base exemplified is one which is exemplified just for convenience, and is not intended to pose any limitation.

The template nucleic acid 121 contains a capture sequence "a", and also contains a sequence "b" for labeling on the 5' side of the capture sequence.
By means of this capture sequence "a", the template nucleic acid 121 can be hybridized with the seed probe nucleic acid 122. Subsequent elongation of the 3' end of the seed probe nucleic acid 122 is accomplished using a 5' side-sequence of the capture sequence "a" of the template nucleic acid 121 as a template. Further, the labeled substrate 123 is hybridized with the base of the sequence "b" for labeling contained in the 5' side of the capture sequence "a" of this template nucleic acid 121, and thus the labeled substrate 123 is incorporated, whereby accomplishing the labeling of a probe nucleic acid.

In the above example, a single base is used as a sequence for labeling, but the sequence for labeling is not limited to one having only a single base and may contain a plurality of consecutive bases or a plurality of intermittent bases.

Such a labeling of a probe nucleic acid, when using a capillary-shaped reaction vessel described above, gives a benefit that the amount of a sample and a reagent required is only small.

According to the embodiment of the invention, a probe nucleic acid can readily be labeled. According to this embodiment, it is possible to conduct a selective labeling of a specific base in a probe nucleic acid having a specific sequence desired, which enables a judgment whether a target sequence is present in a test nucleic acid contained in a test sample or not. As a result, it is possible to conduct a gene expression frequency analysis. A probe nucleic acid which is labeled by a method described above is also encompassed in the invention.

### Example 12: Preparation of labeled probe nucleic acid 2

Using the reaction vessel described in Example 1, a labeled probe nucleic acid was prepared similarly to the method described in Example 11 except for changing the elongation reaction solution.

Instead of the elongation reaction solution used in Example 11, an elongation solution {10 mM Tris-HCl (pH 7.5), 7 mM MgCl₂, 0.1 mM DTT, Klenow Fragment (DNA polymerase I, Large Fragment; TOYOBO) at 0.4 units/µL, 10 µM dATP, 10 µM dCTP, either 0 µM dGTP or 10 µM ddGTP, and 10 µM Cy3-dUTP} was used. A labeled probe nucleic acid was prepared under a condition otherwise similar to that in Example 11.

As a result of this reaction, the RV nucleic acid as a seed probe was elongated from its 3' end up to Cy3-U, when 0 µM dGTP was employed. In this case, no further "G", "C" or "A" was elongated. When using 10 µM ddGTP, the RV nucleic acid was elongated from its 3' end up to "G". In this case, no further "C" or "A" was elongated.

Table 5 shows relative values regarding the highest fluorescent intensity as 1 in the presence or absence of Klenow Fragment in the case of 10 nM or 1 nM of a deposition level of the seed probe nucleic acid on the substrate.

**Table 5**

| | | |
|---|---|---|
| Concentration of spot-deposited target | 10 nM | 1 nM |
| Addition of Klenow Fragment | 1.000 | 0.356 |
| No addition of Klenow Fragment | 0.024 | 0.014 |

As shown in Table 5, the absence of Klenow Fragment during the elongation reaction resulted in an extremely low relative value. Accordingly, it was proven that the binding of the labeled nucleic acid to the seed probe nucleic acid is not non-specific but is a result of the elongation by the polymerase. The relative fluorescent intensity was also increased in a manner dependent on the increase in the deposition level of the seed probe nucleic acid.

The invention allows a seed probe nucleic acid hybridized with a template nucleic acid to be labeled exclusively. The labeling level and the elongation degree of the seed probe nucleic acid can also be controlled, when a nucleotide which prevents a further elongation reaction (such as ddNTP) is used as a substrate nucleic acid other than the labeled substrate nucleic acid to be hybridized with a template capture sequence, or when a substrate nucleic acid other than the labeled substrate nucleic acid is not allowed to be present in the reaction system.

The amount of a marker substance to be incorporated into a seed probe nucleic acid depends on the amount of a template nucleic acid. Accordingly, when a test nucleic acid in a sample is used as a template nucleic acid, a labeled probe nucleic acid reflecting information of a nucleic acid expressed under a certain condition can be prepared. Also in such a case, since the amount of the template nucleic acid varies depending on the frequency of the expression of the nucleic acid, it is also possible to examine the gene expression frequency of the target nucleic acid employed as a template nucleic acid simultaneously with the preparation of the labeled probe nucleic acid. Such a gene expression frequency analysis can be conducted using a small amount of a sample. Thus, since all processes can be conducted as a series of the processes in a small vessel, the amount of a nucleic acid substrate (such as dNTP) and the amount of a reagent required can be reduced, and a large number of the samples can be analyzed within a short time period.

### Example 13: Preparation of labeled probe nucleic acid 3

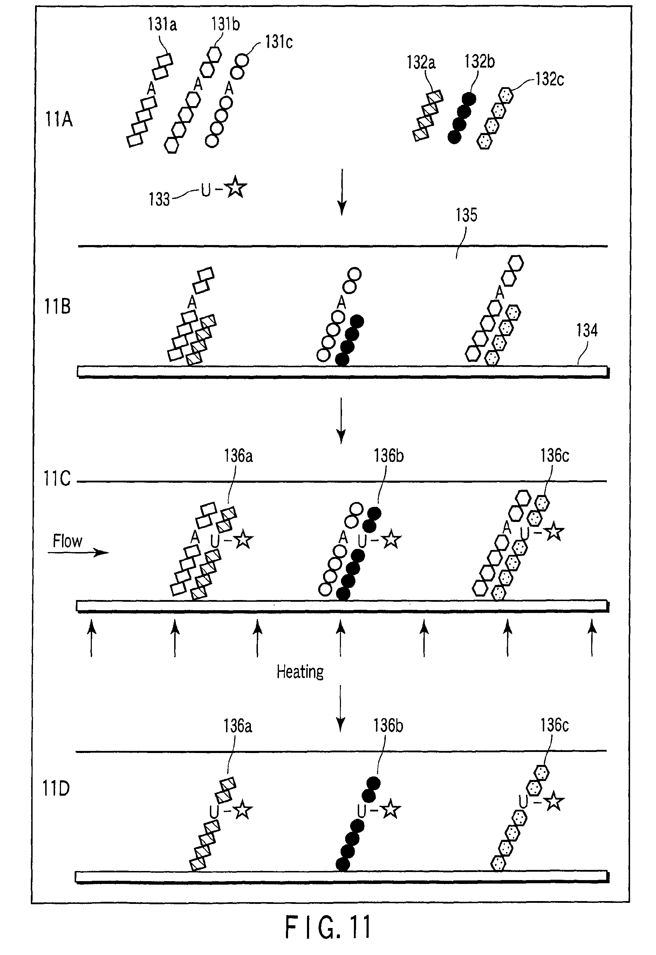

Example 13 is discussed with referring to a schematic view of FIG. 11. In this example, a reaction vessel produced similarly to Example 1 is employed except that a first seed probe nucleic acid 132a, a second seed probe nucleic acid 132b and a third seed probe nucleic acid 132c whose sequences are different from each other are immobilized on a bottom 134 of a reaction zone. In FIG. 11, 11A, a sequence for labeling contained in each of the template nucleic acids 131a to 131c is indicated as "A" for convenience.

The preparation of the labeled probe nucleic acid according to the invention can be conducted as follows. First, as shown in 11B, the template nucleic acids 131a to 131c are hybridized with the seed probe nucleic acids 132a to 132c, respectively, and then subjected to an elongation reaction as shown in 11C using a labeled substrate nucleic acid 133 as a substrate (11B to 11C). Then, the temperature of the reaction zone 135 is elevated for example to about 95°C (11C) to dissociate the template nucleic acid 131 from the elongated probe nucleic acid 136. Then a buffer solution such as 0.1 × SSC is injected into the capillary having the reaction zone 135, and then discharged to let the inner solution flow. As a result, the dissociated target nucleic acid is removed whereby obtaining elongated and labeled probe nucleic acids 136a to 136c each as a single strand (11D).

In the embodiment described above, a heat treatment was conducted as a means for dissociating the double-stranded nucleic acid, but the means for dissociating the double-stranded nucleic acid is not limited to the heat treatment as long as it can be used according to an embodiment of the invention. Thus, any method known per se which is used generally for dissociating a double-stranded nucleic acid into single strands may be employed. For example, an alkaline solution, urea and formamide may be employed.

In the case where the template nucleic acid is an RNA and the probe nucleic acid is a DNA, the dissociation into single strands may be conducted using an enzyme degrading the RNA such as an RNAase H.

In the example, three types of the seed probe nucleic acids and the template nucleic acids was exemplified above, but more or less number of the types of the seed probe nucleic acids and/or template nucleic acids may be employed, and one or more nucleic acids of various types may be employed.

According to an embodiment of the invention, a part of the method described in Examples 9 to 13 may be combined with each other as desired, or a part of the method of Examples 9 to 13 may be modified as desired.

In a conventional method, a highly sophisticated technology is required for labeling a intermediate site of a probe nucleic acid. Also when a probe nucleic acid whose intermediate site is labeled is prepared by a specialty company, an enormous time and expense are required. On the contrary, according to an embodiment of the invention, the elongation reaction of a nucleotide can be controlled. Therefore, a desirable intermediate site of a labeled probe can readily be labeled. Accordingly, a probe nucleic acid to be utilized in a nuclease protection assay can be prepared efficiently within a short period.

### Example 14: Preparation of labeled probe nucleic acid 4

According to a further aspect of the invention, different template nucleic acids can be hybridized with a single seed probe nucleic acid a plurality of times repetitively to effect an elongation.

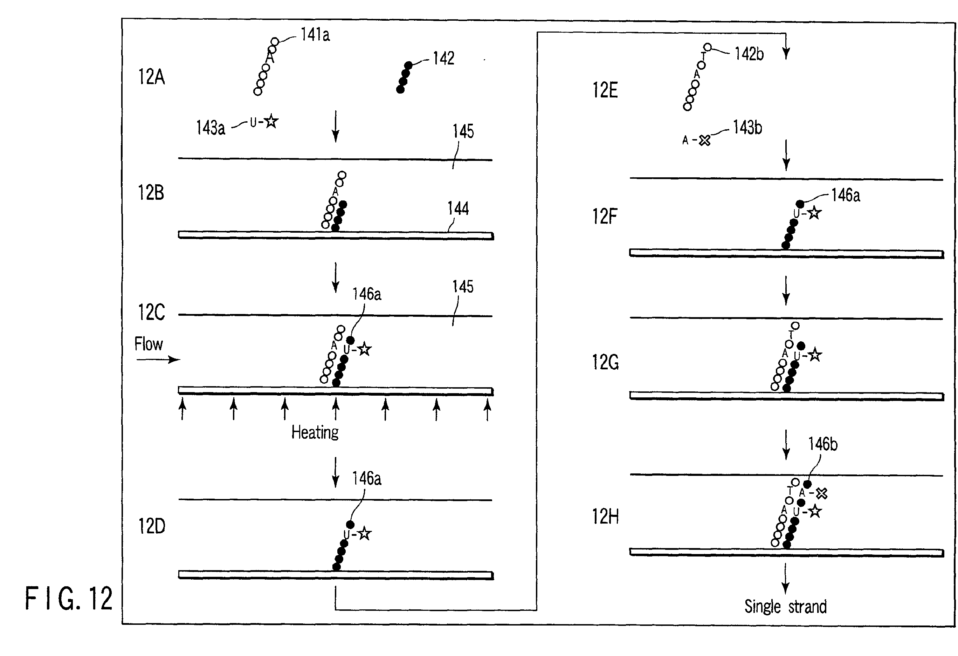

A case in which the labeling is repeated twice according to an embodiment of the invention using the same reaction vessel as that described in Example 1 is discussed with referring to FIG. 12.

First, first template nucleic acid 141, a seed probe nucleic acid 142 and a fluorescent labeled substrate nucleic acid 143a are prepared (12A). Then, a seed probe nucleic acid 142 is immobilized on a bottom 144 of a reaction zone 145 (12B). Then, to the reaction zone 145, a first template nucleic acid 141a, a first fluorescent labeled substrate nucleic acid 143a (a labeled dUTP is shown in FIG. 12 as an example) and a non-labeled substrate nucleic acid (such as dCTP, dGTP and dATP; they are not shown in FIG. 12) are added under a condition enabling a hybridization reaction whereby effecting the hybridization (12B). Then, a polymerase is added to conduct an elongation reaction up to the base next to the first sequence for labeling (indicated as "A" in FIG. 12) (12C).

In order to arrest the elongation reaction at a desired site, i.e., "the base next to the first sequence for labeling", a dNTP having a base complementary to a further next base to "the base next to the first sequence for labeling" is not added. Alternatively, as a nucleotide substrate having a base complementary to "the base next to the first sequence for labeling", a ddNTP may be employed.

In the case described above, the florescent labeled substrate nucleic acid 143a and the non-labeled substrate nucleic acid were added before the hybridization, but the addition of the florescent labeled substrate nucleic acid 143a and the non-labeled substrate nucleic acid may be conducted after the hybridization, i.e., simultaneously with, or before or after the polymerase addition.

Subsequently, as shown in 12C, the temperature of the reaction zone 145 filled with a 0.1 × SSC solution is raised to 95°C to dissociate the target nucleic acid 141a from the elongated probe nucleic acid 146a. The dissociated nucleic acid is removed by injecting a solution such as 0.1 × SSC into the capillary having the reaction zone 145 and discharging it from the capillary to let the inner solution flow. As a result, as shown in 12D, a elongated and labeled probe nucleic acid 146a is obtained as a single strand (FIG. 12).

Subsequently, as shown in 12E, a second template nucleic acid 142b, a second fluorescent labeled substrate nucleic acid 143b (a labeled dATP is shown in FIG. 12 as an example) and a non-labeled substrate nucleic acid (such as dCTP, dGTP and dUTP; they are not shown in FIG. 12) are added under a condition enabling a hybridization reaction whereby effecting the hybridization (12G). Then, a polymerase is added to conduct an elongation reaction up to the base next to the second sequence for labeling (indicated as "T" in FIG. 12) (12H). Then, the template nucleic acid 142b is dissociated to obtain a single strand.

In the case described above, the second florescent labeled substrate nucleic acid 143b and the non-labeled substrate nucleic acid were added before the hybridization, but the addition may be conducted after the hybridization, i.e., simultaneously with, or before or after the polymerase addition.

In this embodiment, the elongation reaction was effected up to the base next to the second sequence for labeling, but a further elongation reaction may be effected or the elongation reaction may be terminated at the second target sequence.

In this embodiment, as a result of the seed probe nucleic acid elongation, a single base was located between the first sequence for labeling and the second sequence for labeling, but it is not limitative, and the base may not be located between the both sequence for labeling, or two or more bases may be located between the both sequence for labeling.

As shown in 12H, the first fluorescent substance (indicated by star in FIG. 12) and the second fluorescent substance (indicated by X in FIG. 12) contained in the second elongated and labeled probe nucleic acid 146b thus obtained are preferably distinguishable, and preferably they emit fluorescence at wavelengths different from each other. The wavelengths may not always be different depending on the selection of the detecting means, as in a case of the judgment based on the difference in the fluorescent intensity.

The second elongated and labeled probe nucleic acid 146b thus obtained may be converted into a single strand and subjected to a further repeat of the method described above.

One typical example is described below.

First, using a reaction vessel similar to the reaction vessel described in Example 1, a first seed probe nucleic acid is hybridized with a first template nucleic acid under a condition enabling a hybridization reaction. Subsequently, an elongation reaction is conducted by a method similar to that in Example 11 except for using an elongation solution {10 mM Tris-HCl (pH 7.5), 7 mM MgCl₂, 0.1 mM DTT, Klenow Fragment (DNA polymerase I, Large Fragment; TOYOBO) at 0.4 units/µL, 10 µM dATP, 10 µM dCTP, 0 µM dGTP and 10 µM Cy3-dUTP}.

As a result of the hybridization and elongation reaction, the presence of the first template nucleic acid and its sequence information are reflected on the elongation and the labeling of the first probe nucleic acid. Specifically, the first seed probe nucleic acid is elongated using as a template the first template nucleic acid hybridized with the first seed probe nucleic acid, whereby incorporating the Cy3-U and being elongated till before "G" (i.e., dGTP). As a result, a first elongated and labeled probe nucleic acid hybridized with the first template nucleic acid is obtained.

Then, by elevating the temperature to about 95°C, the first template nucleic acid is dissociated from the first elongated labeled probe nucleic acid. Furthermore, a dissociated first template nucleic acid is removed by letting the inner solution contained in the reaction zone flow.

Subsequently, the first elongated labeled probe nucleic acid is hybridized with a second template nucleic acid under a condition enabling a hybridization reaction. Then, an elongation reaction is conducted by a method similar to that in Example 11 except for using an elongation solution {10 mM Tris-HCl (pH 7.5), 7 mM MgCl₂, 0.1 mM DTT, Klenow Fragment (DNA polymerase I, Large Fragment; TOYOBO) at 0.4 units/µL, 10 µM Cy5-dATP, 0 µM dCTP, 10 µM dGTP and 10 µM dTTP}.

As a result of the reaction described above, the presence of the second template nucleic acid is reflected on the further elongation and the further labeling. Specifically, the first elongated labeled probe nucleic acid is hybridized with the second template nucleic acid, followed by a further elongation and labeling, so that a second elongated labeled probe nucleic acid is obtained. The second elongated labeled probe nucleic acid is labeled with Cy5-A and elongated till before "C" (i.e., dCTP).

Then, by denaturing the resultant double strand into single strands, a labeled probe nucleic acid can be obtained. This labeled probe nucleic acid can be used after recovery from the substrate if desired.

It is also possible to measure the amount or the relative amount of the first template nucleic acid and the second template nucleic acid by means of measuring the fluorescent intensities of the Cy3 and the Cy5 independently or relatively.

According to the embodiment of the invention, it becomes possible to conduct all processes with regard to plural times of hybridization and labeling in a small vessel all at once, i.e., in a series of procedures. Accordingly, a desired gene expression frequency analysis can be conducted with a small amount of a sample within a short time period in a convenient manner. In addition, the degree of the elongation of the probe nucleic acid can be controlled, and the labeling can readily be conducted at a desired site halfway in the course of the elongation of a desired probe nucleic acid.

### Example 15: Preparation of labeled probe nucleic acid 5

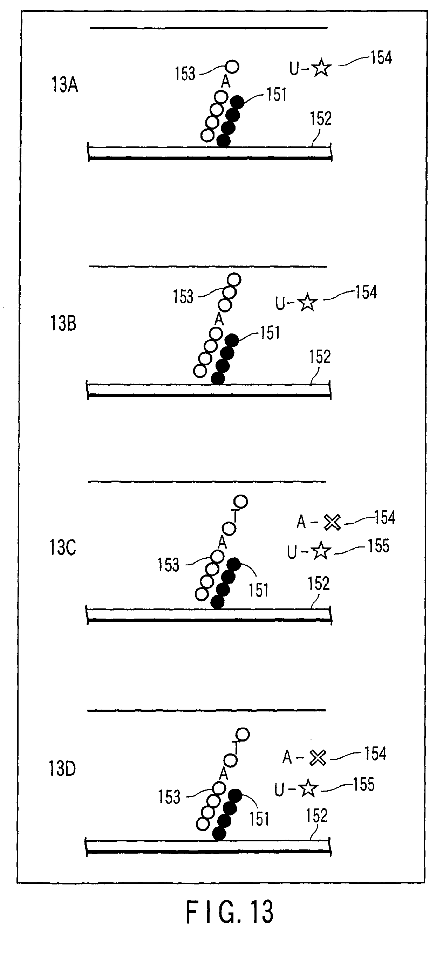

Several examples of a state in which a seed probe nucleic acid is hybridized with a template nucleic acid are illustrated schematically in 13A to 13D of FIG. 13.

As described above, a seed probe nucleic acid 151 is immobilized via its 5' end on a bottom 152 of a reaction zone.

As shown in 13A, the entire length of a template nucleic acid 153 is preferably longer than the entire length of the seed probe nucleic acid 151.
As a result, when the seed probe nucleic acid 151 is hybridized with the template nucleic acid 153, the 3' end of the seed probe nucleic acid 151 is elongated using as a template a single strand part of the template nucleic acid 153 derived from a difference in the length of both nucleic acid. The sequence for labeling is present preferably closer to the 3' end in the single strand part of the template nucleic acid 153.

Also as shown in 13A and 13C, a sequence complementary to the entire length of the seed probe nucleic acid 151 may be contained on the 3' side of the template nucleic acid 153, and a further additional sequence may be contained on the 5' side of the template nucleic acid.

Alternatively, as shown in 13B and 13D, a sequence complementary to a part of the seed probe nucleic acid 151 may be contained on the 3' side of the template nucleic acid 153, and a further additional sequence may be contained on the 5' side of the template nucleic acid 153.

Also as shown in 13A and 13B, a single nucleotide as a sequence for labeling may be provided in a single position, and as shown in 13C and 13D a single nucleotide as a sequence for labeling may be provided in two or more positions.

A labeled probe nucleic acid obtained as described above or from Examples 11 to 15 may be used for a similar nuclease protection assay in Examples 9 and 10. A single-stranded labeled probe nucleic acid obtained by Examples 11 to 15 may be used in a nuclease protection assay as being immobilized on a substrate, or may be utilized after isolation and recovery from the substrate. It may be used also after recovery followed by purification.

By using a labeled probe nucleic acid obtained as described above, a nuclease protection assay can be performed with an advantage described below. Specifically, according to the invention, the probe nucleic acid can be labeled with a fluorescent substance in advance, and therefore the level of the immobilized probe nucleic acid can be known before hybridization. Thus, it is possible to know the immobilized probe level or the deposited spot condition before a reaction. Accordingly, it is easy to control the accuracy of the immobilization of the probe nucleic acid. In addition, in the case of using an mRNA as a sample, it can be directly subjected to hybridization unlike a conventional method, and therefore it is possible to avoid any reduction of the efficiency resulting from the procedure such as cDNA preparation using a reverse transcriptase or labeling.

According to the invention described above, an efficient gene expression frequency analysis method is provided. Also according to the invention described above, a gene expression frequency analysis method enabling a comparison between a plurality of plates is provided. Furthermore, the invention provides a gene expression frequency analysis method having an excellent detection accuracy.

### 3. Method for producing support having labeled probe nucleic acid immobilized thereon

### 3.1 Outlines of method for producing support having labeled probe nucleic acid immobilized thereon

The "method for preparing labeled probe nucleic acid" discussed in the section 2.2 described above can be referred to as a method for producing a support having a labeled probe nucleic acid immobilized thereon according to another aspect. A method for producing a support having a labeled probe nucleic acid immobilized thereon is detailed below. A support having a labeled probe nucleic acid immobilized thereon that is produced according to this method is useful in various analyses using nucleic acids, for example a gene expression analysis. It is useful especially in the "gene expression analysis method utilizing nuclease protection assay" discussed in the section 2.1 described above.

A method for producing a support having a labeled probe nucleic acid immobilized thereon is described below while exemplifying first to third embodiments.

### First embodiment

A method according to a first embodiment of the invention is a method for preparing a support having a labeled probe nucleic acid immobilized thereon comprising:
(1) a step of reacting between a template nucleic acid complementary to the nucleotide sequence of a labeled probe nucleic acid to be prepared and an unlabeled probe nucleic acid precursor which is immobilized on a substrate via its 5' end, under a condition for allowing them to be hybridized, wherein the unlabeled probe nucleic acid precursor has a fewer total number of the bases than the template nucleic acid and has a deletion of one or more nucleotides at the 3' end of the labeled probe nucleic acid to be prepared,;
(2) a step of elongating the deficient part of the unlabeled probe nucleic acid precursor in the direction from 5' to 3', using both a labeled nucleotide of a specific base to which a marker substance generating a detectable signal is attached and non-labeled nucleotides of bases other than the specific base as substrates and using the template nucleic acid as a template, whereby synthesizing a labeled probe nucleic acid;
(3) a step of dissociating all complementary bonds between the labeled probe nucleic acid obtained in the step described above and the template nucleic acid; and
(4) a step of removing the dissociated template nucleic acid from the support.

In the first embodiment, each labeled probe nucleic acid produced on a support contains a labeled nucleotide on its 3' side.

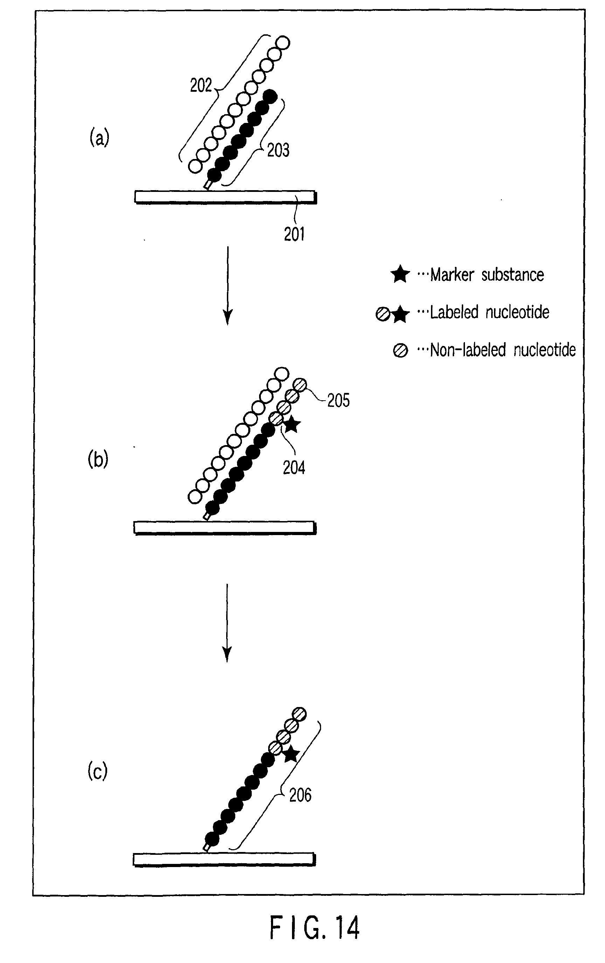

Each step is described in order with referring to FIG. 14.

### [Step (1)]

By the reaction of the step (1), a condition shown schematically in FIG. 14 (a) is established.

First, depending on an intended analysis, a nucleotide sequence of a labeled probe nucleic acid to be formed on a support is designed. Generally, the length of a labeled probe nucleic acid can be of 15 to 50 bases.

Based on the nucleotide sequence of the designed labeled probe nucleic acid, a "template nucleic acid 202" and an "unlabeled probe nucleic acid precursor 203" are prepared.

Here the "template nucleic acid 202" and an "unlabeled probe nucleic acid precursor 203" can consist respectively of DNA-constituting deoxyribonucleotides (dATP, dTTP, dGTP, dCTP), RNA-constituting ribonucleotides (ATP, UTP, GTP, CTP) and other nucleotides such as dUTP.

The "template nucleic acid 202" is prepared so that it has a sequence complementary to a labeled probe nucleic acid to be prepared. On the other hand, the "unlabeled probe nucleic acid precursor 203" is prepared so that it has a fewer total number of the bases than the template nucleic acid and has a deletion of one or more nucleotides at the 3' end of the labeled probe nucleic acid to be prepared. Into this deficient part, at least one labeled nucleotide is introduced in the later step. Here the number of nucleotides to be deleted is preferably 2 to 10. The incorporation of a label into a nucleotide at the 3' end of the labeled probe nucleic acid should be avoided in view of the stability of the label, and therefore the number of nucleotides to be deleted is preferably 2 or more. Further, the number of the labeled nucleotides to be incorporated should be limited, and therefore the number of nucleotides to be deleted is preferably 10 or less.

The number of nucleotides to be deleted is determined taking into consideration the nucleotide sequence on the 3' side of the labeled probe nucleic acid to be prepared and the type of the base of the labeled nucleotide to be introduced into the deficient part. For example, when a labeled probe nucleic acid to be prepared has a sequence AACUACAUUA · · · from its 3' end and the labeled nucleotide to be introduced into the deficient part is a Cy5-dUTP, then the "unlabeled probe nucleic acid precursor" is allowed to have a deletion of 4 to 7 nucleotides from the 3' end of the labeled probe nucleic acid to be prepared. In such a case, only a single labeled nucleotide Cy5-dTP is introduced into the deficient part. Thus, by controlling the number of the nucleotide to be deleted so that only a single labeled nucleotide is introduced into the "unlabeled probe nucleic acid precursor", all labeled probes on a DNA array can uniformly be labeled with identical amounts of the marker substance.

The "unlabeled probe nucleic acid precursor 203" is immobilized at its 5' end on a support 201 (FIG. 14(a)). In a method of the invention, the "support 201" may be in any form as long as it enables a hybridization reaction thereon. For example, it may be a generally employed reaction vessel, including a reaction vessel having a reaction zone for conducting a reaction therein such as a capillary or well, or may be a plate or spherical substrate for conducting the reaction on its surface. Alternatively, the "support" may be a needle, strand, fiber, disc or porous filter. A reaction vessel having a reaction zone in the form of a capillary (hereinafter also referred to as a capillary array) is preferred because it is easy to exchange a solution in the reaction zone. For details of a capillary array, see the descriptions of Examples 1 and 2 described above. As used herein, the term "support" has a mean similar to a "substrate" mentioned above.

The immobilization of the "unlabeled probe nucleic acid precursor 203" onto a support may be conducted by a method known to those skilled in the art. For example, the immobilization of a nucleic acid may be conducted, as described in the following Examples, by means of a biotin-avidin reaction between an avidin-coated support and a nucleic acid having a biotin-labeled 5'-end, or by means of a chemical binding between a support into which a reactive group is introduced and a nucleic acid having at its 5'-end another group reactive with the reactive group.

In the step (1), a state in which the "template nucleic acid 202" is hybridized with the "unlabeled probe nucleic acid precursor 203" immobilized on the support 201 is established (see FIG. 14(a)). This hybridization may be conducted by hybridizing a "template nucleic acid" with an "unlabeled probe nucleic acid precursor" which is not immobilized on the support followed by immobilizing the hybrid on the support, or by hybridizing a "template nucleic acid" with a "unlabeled probe nucleic acid precursor" which has previously been immobilized on the support.

The phrase "reacted under a condition for enabling a hybridization reaction" means, for example, allowing to stand in an appropriate hybridization solution (1 × SSC) at 25 to 70°C (or first at 95°C for 5 minutes followed by allowing to cool slowly and then at 25 to 70°C) for 5 to 60 minutes.

### [Step (2)]

In the step (2), the deficient part (i.e., the 3'-end) of a "unlabeled probe nucleic acid precursor 203" is repaired by an elongation reaction using a "template nucleic acid 202" as a template while introducing a labeled nucleotide 204 into the "unlabeled probe nucleic acid precursor 203" (see FIG. 14(b)).

Here the elongation reaction utilizes as its nucleotide substrates a "labeled nucleotide 4 of a specific base labeled with a marker substance generating a detectable signal" and a "non-labeled nucleotide 5 of each base other than this specific base". As used herein, a "marker substance" means a substance capable of generating a detectable signal, for example, fluorescent substance, radioactive substance and chemiluminescent substance. A labeled nucleotide of a single type of a base can be used as a "labeled nucleotide of a specific base", and each nucleotide of other three types of the bases can be used as a "non-labeled nucleotide of each base other than this specific base". However, such a use is not limitative, and nucleotides of a plurality of the base types may be employed as a "labeled nucleotides of a specific base". Alternatively, as described later in the second embodiment, a nucleotide of a particular base type may not be employed as a nucleotide substrate. For example, a Cy5-dUTP can be used as a "labeled nucleotide" and a dATP, dCTP and dGTP can be used as a "non-labeled nucleotide".

As an elongation reaction solution, any known solution containing each nucleotide as a substrate as described above, an elongation reaction enzyme (T4 DNA polymerase), and Tris buffer and Mg²⁺ can be used. The elongation reaction is conducted for example at 25 to 70°C for 5 to 120 minutes.

When a Cy5-dUTP is used as a "labeled nucleotide", the number of the nucleotides in the deficient part of an "unlabeled probe nucleic acid precursor" should be designed so that at least one Cy5-dUTP is introduced by the elongation reaction into the deficient part of the "unlabeled probe nucleic acid precursor". Specifically, the Cy5-dUTP is not introduced unless at least one dATP is present in the part of the "template nucleic acid" serving as a template upon elongation reaction, and therefore the number of the nucleotides in the deficient part of the "unlabeled probe nucleic acid precursor" should be designed so that the Cy5-dUTP is introduced.

Preferably, when a Cy5-dUTP is used as a "labeled nucleotide", the number of the nucleotides in the deficient part of an "unlabeled probe nucleic acid precursor" is designed so that "a single" Cy5-dUTP is introduced by the elongation reaction into the deficient part of the "unlabeled probe nucleic acid precursor". When the number of the nucleotides in the deficient part of every "unlabeled probe nucleic acid precursor" on the support is adjusted so that a single Cy5-dUTP is introduced by the elongation reaction, a single Cy5-dUTP can be introduced uniformly into every single molecule of the probe nucleic acid. As a result, all probe nucleic acids on the support can be labeled with uniform label amounts in a single elongation reaction.

However, in the first embodiment, it is not desirable in view of the stability of the label that a label is introduced into the nucleotide at the 3' end of the labeled probe nucleic acid.

### [Step (3)]

In the step (3), all complementary bonds between the "labeled probe nucleic acid" and the "template nucleic acid" formed in the elongation reaction in the step (2) are dissociated (see FIG. 14(c)).

The dissociation here may be conducted, as described later in Example, by replacing the elongation reaction solution on the support with a 0.1N NaOH solution and reacting in the NaOH solution at room temperature for 5 to 10 minutes, or by maintaining at 90 to 98°C for 5 to 10 minutes whereby effecting a heat treatment.

### [Step (4)]

In the step (4), the "template nucleic acid" dissociated in the step (3) are removed from the support, and the support on which the labeled probe nucleic acid has been immobilized is produced. The removal may be effected by washing for example with a buffer solution and autoclaved water.

As was discussed above in the first embodiment, by deleting several nucleotides at the 3' end of a probe nucleic acid precursor on a support, a label can be added on the 3' side of the probe nucleic acid. This procedure of the invention is applied to the preparation of a plurality of the labeled probe nucleic acids on a support to delete several nucleotides at the 3' end of every probe nucleic acid precursor on a support, and thereby all probe nucleic acids on the support can be labeled only in a single elongation reaction.

The method of the invention also allows an uniform amount of the label to be uniformly introduced into every probe nucleic acid on the support, by designing the deficient part of every probe nucleic acid on a support so that only a single labeled nucleotide is introduced depending on the base type of the labeled nucleotide.

As a result, every probe nucleic acid on a support can uniformly be labeled only by a single elongation reaction. Thus, the invention is convenient in that it allows all probe nucleic acids on a support to be labeled by a single elongation reaction, and also is excellent in that it enables a control of the amount of a label to be introduced into a nucleic acid probe by controlling the number of nucleotides in a deficient part.

### Second Embodiment

A method of the invention according to a second embodiment is similar basically to the method according to the first embodiment, except for the characteristics described below. Specifically, a method according to the second embodiment is characterized in that a "labeled nucleotide of a specific base" used in the elongation reaction for compensating a deficient part is a labeled nucleotide of a single type of a base, and at least one of the "non-labeled nucleotides of bases other than the specific base" used in the elongation reaction for compensating the deficient part is a dideoxynucleotide or absent.

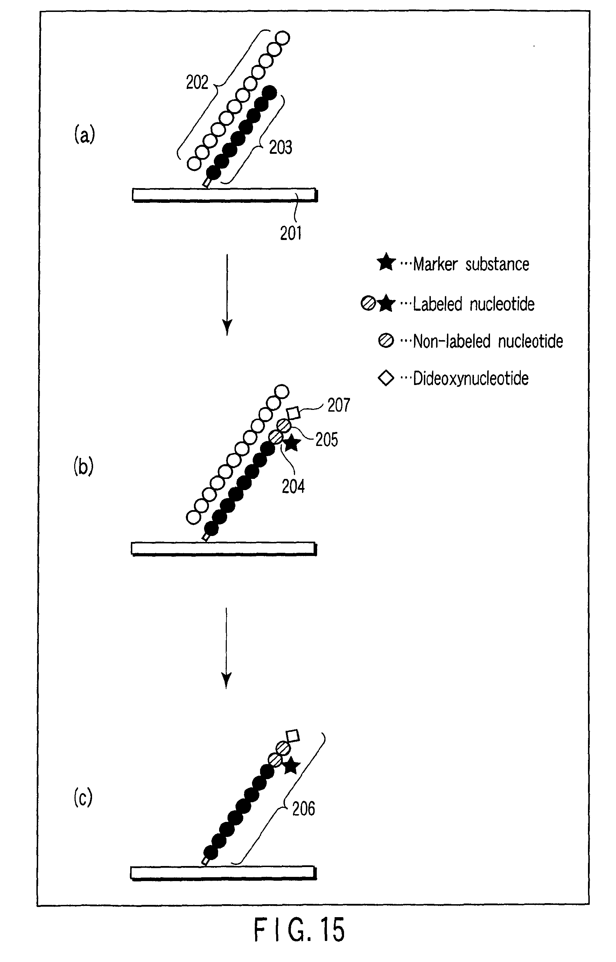

Thus, since the method of the invention according to second embodiment is similar to the method according to the first embodiment except for the above characteristics, details of each step can be understood by referring to the respective description of the steps of the first embodiment. The outlines of the second embodiment are shown schematically in FIG. 15.

In the second embodiment, a state in which the "template nucleic acid 202" is hybridized with the "unlabeled probe nucleic acid precursor 203" immobilized on the support 201 is established (see FIG. 15(a)). Then, the deficient part (i.e., the 3'-end) of a "unlabeled probe nucleic acid precursor 203" is repaired by an elongation reaction using a "template nucleic acid 202" as a template while introducing a labeled nucleotide 204 into the "unlabeled probe nucleic acid precursor 203" (see FIG. 15(b)).

By using a dideoxynucleotide 207 as a "non-labeled nucleotide", the elongation reaction after introduction of the dideoxynucleotide into a deficient part can be terminated. When a Cy5-dUTP is used as a "labeled nucleotide 204", at least one of ddATP, ddCTP and ddGTP can be used as a "dideoxynucleotide 207". For example, when the "unlabeled probe nucleic acid precursor" has a deletion sequence UUAACUA from its 3' end and Cy5-dUTP is used as the labeled nucleotide to be introduced into the deficient part, then ddCTP can be used as a "non-labeled nucleotide" to terminate the elongation reaction halfway through the course. By using a ddCTP as a "non-labeled nucleotide", a single molecule of the Cy5-dUTP is incorporated upon elongation reaction instead of the incorporation of 3 molecules of Cy5-dUTP.

Alternatively, by means of the absence of a nucleotide whose base is a cytosine (dCTP, ddCTP) in an elongation reaction, the elongation reaction can be terminated.

After the elongation reaction, all complementary bonds between the "labeled probe nucleic acid" and the "template nucleic acid" formed in the elongation reaction are dissociated (see FIG. 15(c)).

In the second embodiment, an elongation reaction is terminated by using a dideoxynucleotide or by avoiding a use of a nucleotide of a specific base. Thus, the amount of a marker substance to be introduced into a probe nucleic acid can be controlled by the use of a dideoxynucleotide of a specific base or by the removal of a nucleotide of a specific base.

### Third Embodiment

A method of the invention according to a third embodiment is a method for preparing a support having a labeled probe nucleic acid immobilized thereon, the method comprising:
(1) a step of reacting between a template nucleic acid complementary to the nucleotide sequence of a labeled probe nucleic acid to be prepared and an unlabeled probe nucleic acid precursor which is immobilized on a substrate via its 5' end, under a condition for allowing them to be hybridized, wherein the unlabeled probe nucleic acid precursor has a fewer total number of the bases than the template nucleic acid and has a deletion of a part of the nucleotide sequence in the intermediate part of the labeled probe nucleic acid to be prepared;
(2) a step of elongating the deficient part of the unlabeled probe nucleic acid precursor in the direction from 5' to 3', using both a labeled nucleotide of a specific base to which a marker substance generating a detectable signal is attached and non-labeled nucleotides of bases other than the specific base as substrates and using the template nucleic acid as a template, whereby synthesizing a labeled probe nucleic acid;
(3) a step of dissociating all complementary bonds between the labeled probe nucleic acid obtained in the step (2) and the template nucleic acid; and
(4) a step of removing the dissociated template nucleic acid from the support.

In the third embodiment, each labeled probe nucleic acid formed on a support contains a labeled nucleotide in its intermediate part (for example a center part). Except for this, the third embodiment is similar to the first embodiment.

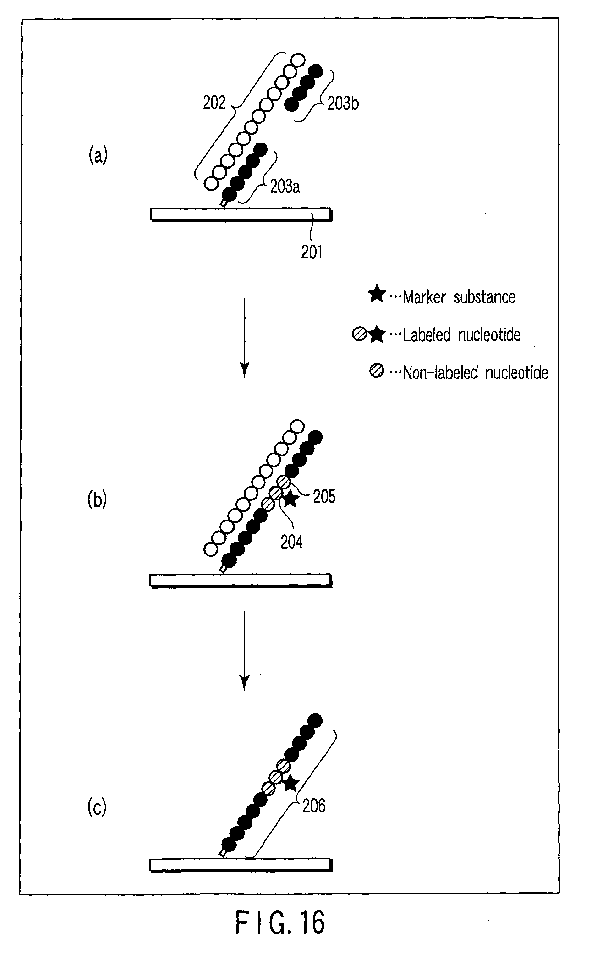

Accordingly, each step of the third embodiment is discussed in order with referring to FIG. 16, and details of the respective steps (1) to (4) can be understood by referring to the descriptions of the steps (1) to (4) of the first embodiment.

### [Step (1)]

By the reaction of the step (1), a condition shown schematically in FIG. 16(a) is established.

Similarly to the first embodiment, a nucleotide sequence of a labeled probe nucleic acid to be formed on a support is designed depending on an intended analysis. A "template nucleic acid 202" is prepared so that it has a sequence complementary to a labeled probe nucleic acid to be prepared. On the other hand, "unlabeled probe nucleic acid precursors 203a and 203b" are prepared so that they have a fewer total number of the bases than the template nucleic acid and has a partial deletion of nucleotides in the intermediate. part of the labeled probe nucleic acid to be prepared.

In this embodiment, since the "unlabeled probe nucleic acid precursor" have a partial deletion of nucleotides in the intermediate part of the labeled probe nucleic acid to be prepared as shown in FIG. 16(a), it consists of two "unlabeled probe nucleic acid precursors 203a and 203b" interrupted by the deficient part. The two "unlabeled probe nucleic acid precursors 203a and 203b" are referred to as the "unlabeled probe nucleic acid precursor A" and the "unlabeled probe nucleic acid precursor B" respectively in FIG. 16. Each of the "unlabeled probe nucleic acid precursor A" and the "unlabeled probe nucleic acid precursor B" should have a length capable of hybridizing independently with a "template nucleic acid". Accordingly, each of the "unlabeled probe nucleic acid precursor A" and the "unlabeled probe nucleic acid precursor B" has a length of at least 6 nucleotides, preferably has a length of 10 to 15 nucleotides. Since a deficient part is formed between two "unlabeled probe nucleic acid precursors" each having a certain length, a "intermediate part" necessarily becomes a center part of the labeled nucleic acid probe to be prepared.

The number of nucleotides to be deleted is generally 1 to 15, preferably 5 to 10. The positions and the number of the nucleotides to be deleted is determined taking into consideration the nucleotide sequence of the labeled probe nucleic acid to be prepared and the type of the base of the labeled nucleotide to be introduced into the deleted region in a later step. For example, when a Cy5-dUTP is used as a labeled nucleotide to be introduced into the deficient part, then the deficient part should contain the position to which a dUTP is introduced. The number of the nucleotides to be deleted may be 1 to 5 adjacent to the dUTP-introduced part in the intermediate part of the labeled probe nucleic acid to be prepared. By selecting the deficient part so that only a single labeled nucleotide Cy5-dUTP is introduced into the deficient part, a uniform amount of the label can uniformly be introduced into every probe nucleic acid on the support.

In the step (1), similarly to the first embodiment, a state in which a "template nucleic acid" is hybridized with an "unlabeled probe nucleic acid precursor" immobilized on a support is established (see FIG. 16(a)).

### [Step (2)]

In the step (2), the deficient part (i.e., the intermediate part) of a "unlabeled probe nucleic acid precursor" is repaired by an elongation reaction using a "template nucleic acid" as a template while introducing a labeled nucleotide 204 between the "unlabeled probe nucleic acid precursor A" and the "unlabeled probe nucleic acid precursor B" (see FIG. 16(b)). The elongation reaction proceeds in the direction from 5' to 3' similarly to the first embodiment. However, in the third embodiment, a nick is present between the 3' end of the last elongated nucleotide and the 5' end of the "unlabeled probe nucleic acid precursor B" (203b in FIG. 16), and therefore a labeled nucleic acid probe is not yet prepared as a complete single strand in this stage by means of only the elongation reaction. Accordingly, after the elongation reaction, it is preferred to conduct a ligation reaction. The ligation reaction is conducted for 30 to 120 minutes at 14 to 37°C in a buffer solution containing an ligase.

The steps (3) and (4) are conducted similarly to the first embodiment to prepare a support having the labeled probe nucleic acid immobilized thereon.

As was discussed above in the third embodiment, by deleting several nucleotides in the intermediate part of a probe nucleic acid precursor on a support, a label can be introduced to the intermediate part of the probe nucleic acid. In the third embodiment, since a label is introduced to the intermediate part of a probe nucleic acid, any position of the intermediate part can be selected as a deficient part. In this context, the third embodiment is excellent in terms of an easier control of the number of labeled nucleotides to be introduced when compared with the first embodiment which limits the deficient part to the 3' end of the probe nucleic acid. The containment of the labeled nucleotide in the intermediate part of the probe nucleic acid is also advantageous because of a stabilization of the label.

In a conventional method, a highly sophisticated technology is required for labeling an intermediate site of a probe nucleic acid. Also, when a probe nucleic acid whose intermediate site is labeled is prepared by a specialty company, an enormous time and expense are required. On the contrary, according to this embodiment, any desired intermediate position of a probe nucleic acid can readily be labeled.

### 3.2 Examples

### Example 16

In Example 16, the 3' end of an "unlabeled probe nucleic acid precursor" is elongated using a labeled dUTP to prepare a "labeled probe nucleic acid".

In this Example, 5'-biotin-TATAAATTCTTTGCTGACCTGCTGGATTAC-3' (SEQ ID No: 6) is used as an "unlabeled probe nucleic acid precursor", and 5'-TTGATGTAATCCAGCAGGTCAGCAAAGAATTTATA-3' (SEQ ID No: 7) is used as a "template nucleic acid".

On a streptavidin-coated slide substrate (Greiner Japan), 20 nM of "unlabeled probe nucleic acid precursor" having biotin bound at its 5' end and 20 nM of "template nucleic acid" were mixed each in an equal amount of 10 µl in a 1 × SSC solution (30 µL), and reacted for 1 hour at 37°C. The "unlabeled probe nucleic acid precursor" and "template nucleic acid" which were hybridized as a result of the reaction were immobilized using a depositing machine on the substrate via the biotin-avidin reaction,

After removing unreacted materials on the substrate by washing, the substrate was covered with a capillary cover having a groove capable of forming a capillary, whereby forming a capillary array onto which a nucleic acid molecule was immobilized. The capillary cover had holes on both ends enabling the access of the solution and had a groove capable of forming a capillary of 3 to 4 cm in length, 1 mm in width and 0.1 to 0.2 mm in depth.

Then, an elongation reaction solution consisting of the following composition was injected into the capillary: 10 mM Tris-HCl (pH 7.5), 7 mM MgCl₂, 0.1 mM DTT, 0.4 units/µL Klenow Fragment (DNA polymerase I, Large Fragment; TOYOBO), 10 µM dATP, 10 µM dCTP, 10 µM dGTP, and 10 µM Cy5-dUTP, and reacted for 1 to 2 hours at 37°C. As a result of the elongation reaction, the "unlabeled probe nucleic acid precursor" on the substrate is elongated at its 3' end in the order of dATP → Cy5-dUTP → dCTP → dATP → dATP, whereby forming a "labeled probe nucleic acid".

After the reaction, any unreacted materials inside of the capillary were removed by washing thoroughly with 1 × SSC. Then, a 0.1 N NaOH alkaline solution was introduced into the capillary, reacted for 5 minutes at room temperature, whereby dissociating the "template nucleic acid" from the "labeled probe nucleic acid". The dissociated "template nucleic acid" was removed from the capillary by washing, whereby forming a capillary array onto which a "labeled probe nucleic acid" was immobilized.

In Table 6, a fluorescent intensity of the "labeled probe nucleic acid" immobilized by the method described above was regarded as 100, and a fluorescent intensity after the same process but in the absence of the "template nucleic acid" (negative control) is indicated as a relative value.

**Table 6**

| | Labeled probe nucleic acid made without template nucleic acid | Labeled probe nucleic acid made with template nucleic acid |
|---|---|---|
| Fluorescent intensity | 0.55 | 100 |

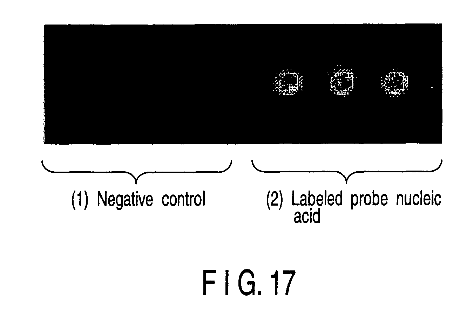

FIG. 17 shows the fluorescent microscopic photograph of the "labeled probe nucleic acid" immobilized by the method described above and the "negative control". In FIG. 17, the "(1) Negative control" exhibited no fluorescence, and the immobilized "(2) Labeled probe nucleic acid" exhibited a fluorescence.

The results shown in Table 6 and FIG. 17 demonstrate that the inventive method enables the production of a labeled probe nucleic acid on a substrate.

### Example 17

In Example 17, similarly to Example 16, the 3' end of an "unlabeled probe nucleic acid precursor" is elongated using a labeled dUTP to prepare a "labeled probe nucleic acid". Example 17 is different from Example 16 in that 0 µM dCTP or 10 µM ddCTP is used instead of 10 µM dCTP to terminate the elongation reaction halfway upon elongation reaction for introducing a labeled dUTP.

A capillary array on which a "labeled probe nucleic acid" is immobilized was produced similarly to Example 16, except for changing the elongation reaction solution employed in Example 16 into the composition shown below. In Example 17, the following elongation reaction solution was used: 10 mM Tris-HCl (pH 7.5), 7 mM MgCl₂, 0.1 mM DTT, 0.4 units/µL Klenow Fragment (DNA polymerase I, Large Fragment; TOYOBO), 10 µM dATP, either 0 µM dCTP or 10 µM ddCTP, 10 µM dGTP, 10 µM Cy5-dUTP.

When using 0 µM dCTP as a substrate nucleotide for the elongation reaction, an "unlabeled probe nucleic acid precursor" on a substrate is elongated at its 3' end in the order of dATP → Cy5-dUTP, and then the reaction is terminated. On the other hand, when using 10 µM ddCTP as a substrate nucleotide for the elongation reaction, the elongation proceeds in the order of dATP → Cy5-dUTP → ddCTP, and then the reaction is terminated. As a result, a "labeled probe nucleic acid" is prepared.

In Table 7, a fluorescent intensity of the "labeled probe nucleic acid" immobilized by the method described above was regarded as 100, and a fluorescent intensity after the same process but in the absence of the "template nucleic acid" (negative control) is indicated as a relative value.

**Table 7**

| | Labeled probe nucleic acid made without template nucleic acid | Labeled probe nucleic acid made with template nucleic acid |
|---|---|---|
| Fluorescent intensity | 1.02 | 100 |

The results shown in Table 7 demonstrate that the inventive method enables the production of a labeled probe nucleic acid on a substrate.

### Example 18

In Example 18, a deficient part located near the center of an "unlabeled probe nucleic acid precursor" is elongated using a labeled dUTP to prepare a "labeled probe nucleic acid".

In this Example, as shown in FIG. 16, two types of oligonucleotides, namely an "unlabeled probe nucleic acid precursor A": 5'-biotin-CAGCAGGTCAGCAAAGAATTT -3' (SEQ ID No: 8) and an "unlabeled probe nucleic acid precursor B": 5'-AGCCCCCCTTGAGCACACAGAGGGCTA -3' (SEQ ID No: 9) were used as "unlabeled probe nucleic acid precursor". As a "template nucleic acid", 5'-TAGCCCTCTGTGTGCTCAAGGGGGGCTATAAATTCTTTGCTGACCTGCTG-3' (SEQ ID No: 10) was used.

On a streptavidin-coated slide substrate (Greiner Japan), 20 nM of "unlabeled probe nucleic acid precursor A", 20 nM of "unlabeled probe nucleic acid precursor B" and 20 nM of "template nucleic acid" were mixed each in an equal amount of 10 µl in a 1 × SSC solution (30 µL), and reacted for 1 hour at 37°C. The "unlabeled probe nucleic acid precursor" and "template nucleic acid" which were hybridized as a result of the reaction were immobilized using a depositing machine on the substrate via the biotin-avidin reaction,

After removing unreacted materials on the substrate by washing, the substrate was covered with a capillary cover having a groove capable of forming a capillary, whereby forming a capillary array onto which a nucleic acid molecule was immobilized. The capillary cover had holes on both ends enabling the access of the solution and had a groove enabling the formation of a capillary of 3 to 4 cm in length, 1 mm in width and 0.1 to 0.2 mm in depth.

Then, an elongation reaction solution consisting of the following composition was injected into the capillary: 10 mM Tris-HCl (pH 7.5), 7 mM MgCl₂, 0.1 mM DTT, 0.4 units/µL Klenow Fragment (DNA polymerase I, Large Fragment; TOYOBO), 10 µM dATP, 10 µM dCTP, 10 µM dGTP, 10 µM Cy5-dUTP, and reacted for 1 to 2 hours at 37°C. As a result of the elongation reaction, the "unlabeled probe nucleic acid precursor A" on the substrate is elongated at its 3' end in the order of dATP → Cy5-dUTP. Thereafter, the nick between the Cy5-dUTP at the 3' end of the "unlabeled probe nucleic acid precursor A" and the dATP at the 5' end of the "unlabeled probe nucleic acid precursor B" was ligated using a ligase. Specifically, the nick was ligated by a reaction for 1 hour at 16°C in a solution of 66 mM Tris-HCl (pH 7.6), 6.6 mM MgCl₂, 10 mM DTT, 0.1 mM ATP, and 100 unites T4 DNA ligase. As a result, a "labeled probe nucleic acid was prepared.

After the reaction, any unreacted materials inside of the capillary were removed by washing thoroughly with 1 × SSC. Then, a 0.1N NaOH alkaline solution was introduced into the capillary, reacted for 5 minutes at room temperature, whereby dissociating the "template nucleic acid" from the "labeled probe nucleic acid". The dissociated "template nucleic acid" was removed from the capillary by washing, whereby forming a capillary array onto which a "labeled probe nucleic acid" was immobilized.

In Table 8, a fluorescent intensity of the "labeled probe nucleic acid" immobilized by the method described above was regarded as 100, and a fluorescent intensity after the same process but in the absence of the "template nucleic acid" (negative control) is indicated as a relative value.

**Table 8**

| | Labeled probe nucleic acid made without template nucleic acid | Labeled probe nucleic acid made with template nucleic acid |
|---|---|---|
| Fluorescent intensity | 0.82 | 100 |

The results shown in Table 8 demonstrate that the inventive method enables the production of a labeled probe nucleic acid on a substrate.

As detailed above, a support having a labeled probe nucleic acid immobilized thereon that is produced according to an inventive method can be utilized in various analysis using such a labeled probe nucleic acid, for example in a gene expression analysis.

An inventive method for labeling a probe nucleic acid on a support is excellent in terms of the aspects listed below, and can solve the problems of conventional sample labeling.

First, since a method of the invention involves no labeling of a sample, there is no possibility of any degradation of the sample. In addition, the inventive method is convenient since it can add labels to all probe nucleic acids on the support at once by a single elongation reaction.

Further, a designed probe having a known sequence is labeled with a marker substance on an array in the invention, and thus the position of the introduction of the marker substance and the labeling efficiency can be controlled. Since an inventive method can introduce a marker substance into any nucleotide in an intermediate part of a probe nucleic acid, it can produce a stable labeled probe so that the label is not released from the probe nucleic acid upon subsequent detection reaction. Moreover, since an inventive method can introduce a marker substance into a specific nucleotide of a probe nucleic acid, a uniform amount of the marker substance can be introduced into every probe on a support. By means of such a labeling of the probe with a uniform amount of the label, a reliable measurement data can be obtained, without being affected by the label amount, when compared with a prior art of uneven labeling of a sample.

Furthermore, an expression analysis can be automated by providing an array on which a labeled probe is immobilized according to the invention. In addition, the gene expression level can be measured as an absolute value of the gene expression level of a sample itself, unlike a competitive hybridization with other sample. Moreover, since a probe on an array can be labeled before a reaction in the present invention, the immobilization state of the probe can be monitored, and therefore such labeling of the probe on an array is desirable in controlling the accuracy of the analysis.

## Claims

1. A method for determining the type of a base at a target site in a target nucleic acid, comprising:
(1) a step of reacting a target nucleic acid sample with a probe nucleic acid, which contains a selection sequence complementary to the nucleotide sequence located on the 3' side adjacent to the target site of the target nucleic acid and which is immobilized on a substrate via its 5' end, in a reaction system under a condition for achieving an appropriate hybridization;
(2) a step of elongating a reaction product obtained in the step (1), in the presence of both a polymerase and a labeled deoxyribonucleoside triphosphate containing a specific one type of base labeled with a marker substance generating a detectable signal, under a condition for obtaining an appropriate elongation reaction;
(3) a step of removing the labeled deoxyribonucleoside triphosphate which has not been used for the elongation reaction in the elongation step (2) from the reaction system;
(4) a step of detecting a signal derived from the marker substance present in the reaction system after the step (3); and
(5) a step of determining the type of a base at the target site based on the presence or absence of the detection of the signal in the step (4) and on the type of the base contained in the labeled deoxyribonucleoside triphosphate.

2. A method for determining the type of a base at a target site in a target nucleic acid, comprising:
(1) a step of reacting a target nucleic acid sample with a probe nucleic acid, which contains a selection sequence complementary to the nucleotide sequence located on the 3' side adjacent to the target site of the target nucleic acid and which is immobilized on a substrate via its 5' end, in a first reaction system and a second reaction system respectively, under a condition for achieving an appropriate hybridization;
(2) a step of elongating a reaction product contained in the first reaction system obtained in the step (1), in the presence of both a polymerase and a labeled deoxyribonucleoside triphosphate containing a first type of base labeled with a marker substance generating a detectable signal, under a condition for obtaining an appropriate elongation reaction;
(3) a step of elongating a reaction product contained in the second reaction system obtained in the step (1), in the presence of both a polymerase and a labeled deoxyribonucleoside triphosphate containing a second type of base labeled with a marker substance generating a detectable signal, under a condition for obtaining an appropriate elongation reaction;
(4) a step of removing the labeled deoxyribonucleoside triphosphate which has not been used for the elongation reaction in the elongation steps (2) and (3) from each reaction system, respectively;
(5) a step of detecting a signal derived from the marker substance present in the first reaction system and the second reaction system, respectively, after the step (4); and
(6) a step of determining the type of a base at the target site based on the presence or absence of the detection of the signal in the step (5) and on the type of the base contained in the labeled deoxyribonucleoside triphosphate.

3. A method for determining the type of a base at a target site in a target nucleic acid, comprising:
(1) a step of reacting a target nucleic acid sample with a probe nucleic acid, which contains a selection sequence complementary to the nucleotide sequence located on the 3' side adjacent to the target site of the target nucleic acid and which is immobilized on a substrate via its 5' end, in a first reaction system, a second reaction system, a third reaction system and a fourth reaction system respectively, under a condition for achieving an appropriate hybridization;
(2) a step of elongating a reaction product contained in the first reaction system obtained in the step (1), in the presence of both a polymerase and a labeled deoxyribonucleoside triphosphate containing a first type of base labeled with a marker substance generating a detectable signal, under a condition for obtaining an appropriate elongation reaction;
(3) a step of elongating a reaction product contained in the second reaction system obtained in the step (1), in the presence of both a polymerase and a labeled deoxyribonucleoside triphosphate containing a second type of base labeled with a marker substance generating a detectable signal, under a condition for obtaining an appropriate elongation reaction;
(4) a step of elongating a reaction product contained in the third reaction system obtained in the step (1), in the presence of both a polymerase and a labeled deoxyribonucleoside triphosphate containing a third type of base labeled with a marker substance generating a detectable signal, under a condition for obtaining an appropriate elongation reaction;
(5) a step of elongating a reaction product contained in the fourth reaction system obtained in the step (1), in the presence of both a polymerase and a labeled deoxyribonucleoside triphosphate containing a fourth type of base labeled with a marker substance generating a detectable signal, under a condition for obtaining an appropriate elongation reaction;
(6) a step of removing the labeled deoxyribonucleoside triphosphate which has not been used for the elongation reaction in the elongation steps (2)-(5) from each reaction system, respectively;
(7) a step of detecting a signal derived from the marker substance present in the first reaction system, the second reaction system, the third reaction system and the fourth reaction system, respectively, after the step (6); and
(8) a step of determining the type of a base at the target site based on the presence or absence of the detection of the signal in the step (7) and on the type of the base contained in the labeled deoxyribonucleoside triphosphate.

4. A method for determining the type of a base at a target site in a target nucleic acid, comprising:
(1) a step of reacting a target nucleic acid sample with a probe nucleic acid, which contains a selection sequence complementary to the nucleotide sequence located on the 3' side adjacent to the target site of the target nucleic acid and which is immobilized on a substrate via its 5' end, in a reaction system under a condition for achieving an appropriate hybridization;
(2) a step of elongating a double-stranded nucleic acid obtained in the step (1), in the presence of a polymerase as well as a first labeled dideoxyribonucleoside triphosphate containing a first type of base labeled with a marker substance generating a first detectable signal, a second labeled dideoxyribonucleoside triphosphate containing a second type of base labeled with a marker substance generating a second detectable signal, a third labeled dideoxyribonucleoside triphosphate containing a third type of base labeled with a marker substance generating a third detectable signal, a fourth labeled dideoxyribonucleoside triphosphate containing a fourth type of base labeled with a marker substance generating a fourth detectable signal, under a condition for obtaining an appropriate elongation reaction;
(3) a step of removing all of the labeled deoxyribonucleoside triphosphates which have not been used for the elongation reaction in the elongation step (2) from the reaction system;
(4) a step of detecting a signal derived from each marker substance present in the reaction system after the step (3); and
(5) a step of determining the type of a base at the target site based on the type of the signal detected in the step (4).

5. A method for determining the type of a base at a target site in a target nucleic acid, comprising:
(1) a step of reacting a target nucleic acid sample with a probe nucleic acid, which contains a selection sequence complementary to the nucleotide sequence located on the 3' side adjacent to the target site of the target nucleic acid and which is immobilized on a substrate via its 5' end, in a reaction system under a condition for achieving an appropriate hybridization;
(2) a step of elongating a double-stranded nucleic acid obtained in the step (1), in the presence of a polymerase, as well as a labeled deoxyribonucleoside triphosphate containing a first type of base labeled with a marker substance generating a detectable signal, and at least one non-labeled deoxyribonucleoside triphosphate containing a second type of base whose type is different from the first type of base, under a condition for obtaining an appropriate elongation reaction;
(3) a step of removing the labeled deoxyribonucleoside triphosphate which has not been used for the elongation reaction in the elongation step (2) from the reaction system;
(4) a step of detecting a signal derived from the marker substance present in the reaction system after the step (3); and
(5) a step of determining the type of a base at the target site based on the presence or absence of the detection of the signal in the step (4) and on the type of the base contained in the labeled deoxyribonucleoside triphosphate.

6. The method for determining the type of a base at a target site in a target nucleic acid according to any one of claims 1 to 5, **characterized in that** the detectable signal is a fluorescence and the marker substance is a fluorescent substance.

7. The method for determining the type of a base at a target site in a target nucleic acid according to any one of claims 1 to 6, **characterized in that** the selection sequence is of a 10-base to 15-base length.

8. The method for determining the type of a base at a target site in a target nucleic acid according to any one of claims 1 to 7, **characterized in that** the target nucleic acid sample has previously been taken out and prepared from a subject and the target site is a potential variation site.

9. The method for determining the type of a base at a target site in a target nucleic acid according to any one of claims 1 to 8, **characterized in that** the target nucleic acid sample has previously been taken out and prepared from a subject and the target site is a potential single base polymorphism site.

10. The method for determining the type of a base at a target site in a target nucleic acid according to any one of claims 1 to 9, **characterized in that** the probe nucleic acid is immobilized on an internal wall of a reaction vessel capable of conducting a reaction therein for a nucleic acid.

11. The method for determining the type of a base at a target site in a target nucleic acid according to claim 10, **characterized in that** the shape of a reaction zone of the reaction vessel is a capillary shape.

12. A method for detecting the presence of a target sequence in a test nucleic acid, comprising:
(1) a step of reacting the test nucleic acid with a labeled probe nucleic acid, which is immobilized onto a channel allowing the reaction to be conducted there and which has a marker substance attached thereto, under a condition for enabling an appropriate hybridization reaction;
(2) a step of adding, after the step (1), a single-stranded specific nuclease to the channel to conduct a reaction under a condition for allowing an appropriate enzyme reaction to be achieved;
(3) a step of removing, after the step (2), a degradation product of the enzyme reaction from the channel;
(4) a step of detecting a signal from the marker substance contained in the double-stranded nucleic acid in the channel; and
(5) a step of detecting the presence of the target sequence in the test nucleic acid based on a signal detected in the step (4).

13. A method for determining the expression frequency of a target sequence in a subject, comprising:
(1) a step of reacting a sample containing a test nucleic acid derived from a subject with a labeled probe nucleic acid, which is immobilized onto a channel allowing the reaction to be conducted there and which has a marker substance attached thereto, under a condition for enabling an appropriate hybridization reaction;
(2) a step of adding, after the step (1), a single-stranded specific nuclease to the channel to conduct a reaction under a condition allowing an appropriate enzyme reaction to be achieved;
(3) a step of removing, after the step (2), a degradation product of the enzyme reaction from the channel;
(4) a step of detecting, after the step (3), a signal derived from the marker substance contained in the double-stranded nucleic acid in the channel; and
(5) a step of determining the expression frequency of the target sequence in the subject based on a signal detected in the step (4).

14. The method for detecting the presence of a target sequence in a test nucleic acid according to claim 12, using a labeled probe nucleic acid obtained from the following steps:
(1) a step of reacting a seed probe nucleic acid with a template nucleic acid containing a complementary sequence complementary to the seed probe nucleic acid, a sequence for labeling present on the 5' side of this complementary sequence and a sequence for elongating present on the 5' side of the sequence for labeling, under a condition for enabling an appropriate hybridization;
(2) a step of elongating the seed probe nucleic acid contained in a double strand obtained in the step (1), in the presence of a polymerase, a labeled nucleotide substrate which contains a base complementary to the base of the sequence for labeling and which is labeled with a marker substance, and a non-labeled nucleotide substrate having a base complementary to the base contained in the sequence for elongating; and
(3) a step of dissociating the double strand obtained by the elongation in the step (2) to obtain a labeled probe nucleic acid as a single strand.

15. A method for preparing a support having a labeled probe nucleic acid immobilized thereon, comprising:
a step of reacting between a template nucleic acid complementary to the nucleotide sequence of a labeled probe nucleic acid to be prepared and an unlabeled probe nucleic acid precursor which is immobilized on a substrate via its 5' end, under a condition for allowing them to be hybridized, wherein the unlabeled probe nucleic acid precursor has a fewer total number of the bases than the template nucleic acid and has a deletion of a part of the nucleotide sequence of the labeled probe nucleic acid to be prepared;
a step of elongating the deficient part of the unlabeled probe nucleic acid precursor in the direction from 5' to 3', using both a labeled nucleotide of a specific base to which a marker substance generating a detectable signal is attached and non-labeled nucleotides of bases other than the specific base as substrates and using the template nucleic acid as a template, whereby synthesizing a labeled probe nucleic acid;
a step of dissociating all complementary bonds between the labeled probe nucleic acid obtained in the above step and the template nucleic acid; and
a step of removing the dissociated template nucleic acid from the support.

16. The method for preparing a support having a labeled probe nucleic acid immobilized thereon according to claim 15, **characterized in that** the unlabeled probe nucleic acid precursor is deficient in one or more nucleotides at the 3' end of the labeled probe nucleic acid to be prepared.

17. The method for preparing a support having a labeled probe nucleic acid immobilized thereon according to claim 15, **characterized in that** the unlabeled probe nucleic acid precursor is deficient in one or more nucleotides at the intermediate part of the labeled probe nucleic acid to be prepared.

18. The method for preparing a support having a labeled probe nucleic acid immobilized thereon according to any one of claims 15 to 17, **characterized in that** the labeled nucleotide of the specific base is a labeled nucleotide of a base of a single type and the non-labeled nucleotides of bases other than the specific base are respective nucleotides of the bases of three types.

19. The method for preparing a support having a labeled probe nucleic acid immobilized thereon according to claim 15 or 16, **characterized in that** the labeled nucleotide of the specific base is a labeled nucleotide of a base of a single type and at least one of the non-labeled nucleotides of bases other than the specific base is a dideoxynucleotide.

20. The method for preparing a support having a labeled probe nucleic acid immobilized thereon according to claim 15 or 16, **characterized in that** the labeled nucleotide of the specific base is a labeled nucleotide of a base of a single type and at least one of the non-labeled nucleotides of bases other than the specific base is absent.


SEQUENCE LISTING

<110> OLYMPUS CORPORATION

<120> Method for analyzing variation of nucleic acid
      and method for analyzing gene expression

<130> 03S1749P

<140> PCT/JP03/15819
<141> 2003-12-10

<150> JP P2002-358504
<151> 2002-12-10

<150> JP P2002-358505
<151> 2002-12-10

<150> JP P2003-408945
<151> 2003-12-08

<160> 10

<170> PatentIn Ver. 2.0

<210> 1
<211> 19

<212> DNA
<213> Artificial Sequence

<400> 1
ggaaacagct atgaccatg 19

<210> 2
<211> 23
<212> DNA
<213> Artificial Sequence

<400> 2
tgcacatggt catagctgtt tcc 23

<210> 3
<211> 23
<212> DNA
<213> Artificial Sequence

<400> 3
tgcgcatggt catagctgtt tcc 23

<210> 4
<211> 30

<212> DNA

<213> Artificial Sequence

<400> 4

gggggctata aattctttgc tgacctgctg 30

<210> 5

<211> 30

<212> DNA

<213> Artificial Sequence

<400> 5

cagcaggtca gcaaagaatt tatagccccc 30

<210> 6

<211> 30

<212> DNA

<213> Artificial Sequence

<400> 6

tataaattct ttgctgacct gctggattac 30

<210> 7

<211> 35

<212> DNA

<213> Artificial Sequence

<400> 7

ttgatgtaat ccagcaggtc agcaaagaat ttata 35

<210> 8

<211> 21

<212> DNA

<213> Artificial Sequence

<400> 8

cagcaggtca gcaaagaatt t 21

<210> 9

<211> 27

<212> DNA

<213> Artificial Sequence

<400> 9

agcccccctt gagcacacag agggcta 27

<210> 10

<211> 50

<212> DNA

<213> Artificial Sequence

<400> 10

tagccctctg tgtgctcaag gggggctata aattctttgc tgacctgctg 50